# EUROPÄISCHE PATENTSCHRIFT

(11) **EP 1 713 789 B1**
(45) Veröffentlichungstag und Bekanntmachung des Hinweises auf die Patenterteilung: **27.05.2015**
(21) Anmeldenummer: 05701130.6
(22) Anmeldetag: 22.01.2005
(51) Int. Cl.: C07D 333/38, C07D 307/68, A01N 43/08, A01N 43/10

(54) **2-HALOGENFURYL/THIENYL-3-CARBOXAMIDE**
2-HALOFURYL/THIENYL-3-CARBOXAMIDES
2-HALOGENOFURYL/THIENYL-3-CARBOXAMIDES

(30) Priorität: 06.02.2004 DE 102004005785
(43) Veröffentlichungstag der Anmeldung: 25.10.2006
(73) Patentinhaber: Bayer Intellectual Property GmbH, 40789 Monheim am Rhein (DE)
(72) Erfinder: DUNKEL, Ralf, 69001 Lyon (FR); ELBE, Hans-Ludwig, 42329 Wuppertal (DE); GREUL, Jörg Nico, 42799 Leichlingen (DE); HARTMANN, Benoit, 40764 Langenfeld (DE); DAHMEN, Peter, 41470 Neuss (DE); KUCK, Karl-Heinz, 40764 Langenfeld (DE); WACHENDORFF-NEUMANN, Ulrike, 56566 Neuwied (DE)
(74) Vertreter: BIP Patents
(86) Internationale Anmeldenummer: PCT/EP2005/000629
(87) Internationale Veröffentlichungsnummer: WO 2005/075452

(56) Entgegenhaltungen:
- WO-A-02/08197
- WO-A-03/010149
- WO-A-2004/005242
- WO-A-2005/004606
- PATENT ABSTRACTS OF JAPAN Bd. 2002, Nr. 02, 2. April 2002 (2002-04-02) & JP 2001 302605 A (SUMITOMO CHEM CO LTD), 31. Oktober 2001 (2001-10-31) in der Anmeldung erwähnt
- PATENT ABSTRACTS OF JAPAN Bd. 1996, Nr. 11, 29. November 1996 (1996-11-29) & JP 08 176112 A (MITSUI TOATSU CHEM INC), 9. Juli 1996 (1996-07-09) in der Anmeldung erwähnt
- PATENT ABSTRACTS OF JAPAN Bd. 014, Nr. 453 (C-0764), 28. September 1990 (1990-09-28) & JP 02 178259 A (TOKUYAMA SODA CO LTD), 11. Juli 1990 (1990-07-11)
- PATENT ABSTRACTS OF JAPAN Bd. 014, Nr. 197 (C-0712), 23. April 1990 (1990-04-23) & JP 02 040374 A (SUMITOMO CHEM CO LTD), 9. Februar 1990 (1990-02-09)
- PATENT ABSTRACTS OF JAPAN Bd. 014, Nr. 109 (C-0695), 28. Februar 1990 (1990-02-28) & JP 01 313402 A (MITSUBISHI KASEI CORP), 18. Dezember 1989 (1989-12-18)

## Beschreibung

Die vorliegende Erfindung betrifft neue 2-Halogenfuryl/thienyl-3-carboxamide, mehrere Verfahren zu deren Herstellung und deren Verwendung zur Bekämpfung von unerwünschten Mikroorganismen.

Es ist bereits bekannt, dass zahlreiche Carboxamide fungizide Eigenschaften besitzen (vgl. z.B. EP-A 0 737 682, EP-A 0 591 699, EP-A 0 589 301, EP-A 0 545 099, DE-A 24 09 011, DE-A 20 06 472, JP-A 2001-302605, JP-A 10-251240, JP-A 8-176112, JP-A 8-92223 und JP-A 53-72823). So sind bereits zahlreiche Furyl/thienyl-3-carboxamide bekannt geworden, deren Furyl/Thienyl-Ring in 2-Position durch Methyl oder Trifluormethyl substituiert ist. Einzige in 2-Position des Heterocyclus durch Halogen substituierte Carboxamide sind das 2,5-Dichlor-N-{3'-[(methoxyimino)methyl]-biphenyl-2-yl}thiophen-3-carboxamid und das 2,5-Dichlor-N-{4'-[(methoxyimino)methyl]biphenyl-2-yl}thiophen-3-carboxamid (vgl. WO 02/08197). Die Wirksamkeit dieser Stoffe ist gut, lässt aber in manchen Fällen, z.B. bei niedrigen Aufwandmengen zu wünschen übrig.

Neu sind 2-Halogenfuryl/thienyl-3-carboxamide, deren Furyl/Thienyl-Ring außer dem Halogensubstituenten keine weiteren Substituenten trägt.

Es wurden nun neue 2-Halogenfuryl/thienyl-3-carboxamide der Formel (I) 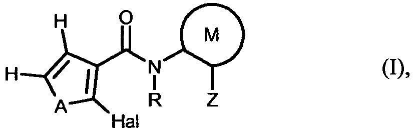 gefunden, in welcher
- A: für O (Sauerstoff) oder S (Schwefel) steht,
- Hal: für Halogen steht,
- R: für Wasserstoff steht,
- M: für einen jeweils einfach durch R⁷ substituierten Phenyl-, Thiophen-, Pyridin-, Pyrimidin-, Pyridazin oder Pyrazin-Ring oder für einen durch R^{7-A} substituierten Thiazol-Ring steht,
- R⁷: für Wasserstoff, Fluor, Chlor, Methyl, iso-Propyl, Methylthio oder Trifluormethyl steht,
- R^{7-A}: für Wasserstoff, Methyl, Methylthio oder Trifluormethyl steht,
- Z: für Z¹, Z², Z³ oder Z⁴ steht, worin
Z¹ für gegebenenfalls einfach bis fünffach, gleich oder verschieden substituiertes Phenyl steht,
Z² für jeweils gegebenenfalls einfach oder mehrfach, gleich oder verschieden substituiertes Cycloalkyl oder Bicycloalkyl steht,
Z³ für unsubstituiertes C₂-C₂₀-Alkyl oder für einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₁-C₂₀-Alkyl steht, wobei der Cycloalkylteil seinerseits gegebenenfalls einfach oder mehrfach, gleich oder verschieden durch Halogen und/oder C₁-C₄-Alkyl substituiert sein kann,
Z⁴ für jeweils gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann,
- R⁸ und R⁹: unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
- R¹⁰: für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht.

Weiterhin wurde gefunden, dass man 2-Halogenfuryl/thienyl-3-carboxamide der Formel (I) erhält, indem man
a) Carbonsäure-Derivate der Formel (II) 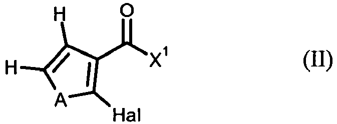 in welcher
   A und Hal die oben angegebenen Bedeutungen haben und
   X¹ für Halogen oder Hydroxy steht,
   mit Anilin-Derivaten der Formel (III) 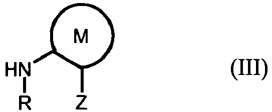 in welcher R, M und Z die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder
b) Halogencarboxamide der Formel (IV) 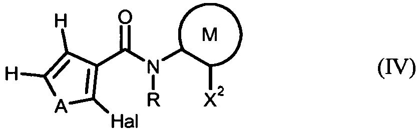 in welcher
   A, Hal, R und M die oben angegebenen Bedeutungen haben,
   X² für Brom, Iod oder Trifluormethylsulfonat steht,
   mit Boronsäure-Derivaten der Formel (V) 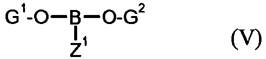 in welcher
   Z¹ die oben angegebenen Bedeutungen hat und
   G¹ und G² jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
c) Boronsäure-Derivate der Formel (VI) 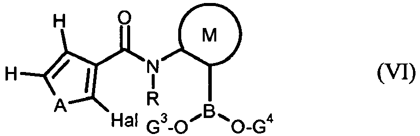 in welcher
   A, Hal, R und M die oben angegebenen Bedeutungen haben,
   G³ und G⁴ jeweils für Wasserstoff oder zusammen für Tetramethylethylen stehen,
   mit Phenyl-Derivaten der Formel (VII)

   X³-Z¹ (VII)

   in welcher
   Z¹ die oben angegebenen Bedeutungen hat und
   X³ für Chlor, Brom, Iod oder Trifluormethylsulfonat steht,
   in Gegenwart eines Katalysators, gegebenenfalls in Gegenwart eines Säurebindemittels und gegebenenfalls in Gegenwart eines Verdünnungsmittels umsetzt, oder
d) Halogencarboxamide der Formel (IV) 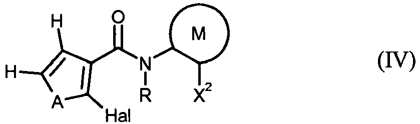 in welcher
   A, Hal, R und M die oben angegebenen Bedeutungen haben,
   X² für Brom, Iod oder Trifluormethylsulfonat steht,
   mit Phenyl-Derivaten der Formel (VII)

   X³-Z¹ (VII)

   in welcher
   Z¹ die oben angegebenen Bedeutungen hat und
   X³ für Chlor, Brom, Iod oder Trifluormethylsulfonat steht,
   in Gegenwart eines Palladium- oder Nickel-Katalysators und in Gegenwart von 4,4,4',4',5,5, 5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt, oder
e) 2-Halogenfuryl/thienyl-3-carboxamide der Formel (I-a) 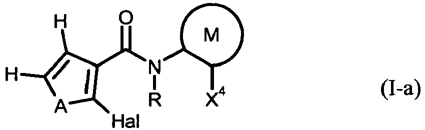 in welcher
   A, Hal, R und M die oben angegebenen Bedeutungen haben,
   X⁴ für jeweils gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart eines Katalysators hydriert, oder
f) Hydroxyalkylcarboxamide der Formel (VIII) 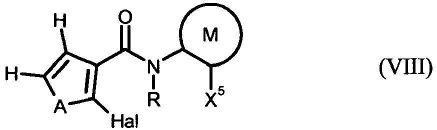 in welcher
   - A, Hal, R und M: die oben angegebenen Bedeutungen haben,
   - X⁵: für gegebenenfalls zusätzlich einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
   gegebenenfalls in Gegenwart eines Verdünnungsmittels und gegebenenfalls in Gegenwart einer Säure dehydratisiert, oder
g) Halogencarboxamide der Formel (IV) 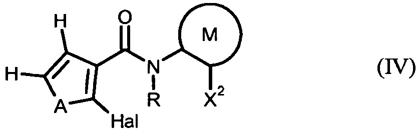 in welcher
   - A, Hal, R und M: die oben angegebenen Bedeutungen haben,
   - X²: für Brom, Iod oder Trifluormethylsulfonat steht,
   mit einem Alkin der Formel (IX) 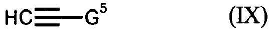 in welcher
   - G⁵: für gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogen-alkylamino, Halogen-dialkylamino, -SiR⁸R⁹R¹⁰ und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
   oder einem Alken der Formel (X) 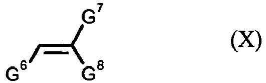 in welcher
   - G⁶, G⁷ und G⁸: unabhängig voneinander jeweils für Wasserstoff oder gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils (ohne die Substituenten) die Zahl 20 nicht übersteigt,
   gegebenenfalls in Gegenwart eines Verdünnungsmittels, gegebenenfalls in Gegenwart eines Säurebindemittels und in Gegenwart eines oder mehrerer Katalysatoren umsetzt, oder
h) Ketone der Formel (XI) 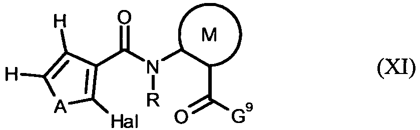 in welcher
   - A, Hal, R und M: die oben angegebenen Bedeutungen haben,
   - G⁹: für Wasserstoff oder gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   mit einer Phosphorverbindung der allgemeinen Formel (XII)

   G¹⁰-Px (XII)

   in welcher
   - G¹⁰: für gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
   - Px: für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃ steht,
   gegebenenfalls in Gegenwart eines Verdünnungsmittels umsetzt, oder.

Schließlich wurde gefunden, dass die neuen 2-Halogenfuryl/thienyl-3-carboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen 2-Halogenfuryl/thienyl-3-carboxamide der Formel (I) eine wesentlich bessere fungizide Wirksamkeit als die konstitutionell ähnlichsten, vorbekannten Wirkstoffe gleicher Wirkungsrichtung.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen 2-Halogenfuryl/thienyl-3-carboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- A: steht bevorzugt für O (Sauerstoff).
- A: steht außerdem bevorzugt für S (Schwefel).

- Hal: steht bevorzugt für Fluor, Chlor, Brom oder Iod.
- Hal: steht besonders bevorzugt, für Chlor, Brom oder Iod.
- Hal: steht ganz besonders bevorzugt für Iod.

- M: steht bevorzugt für einen der folgenden Cyclen 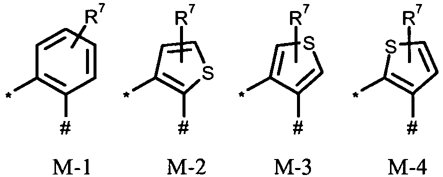 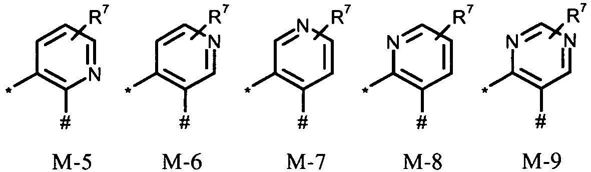 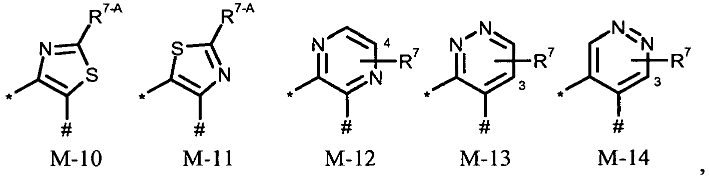 wobei die mit "*" markierte Bindung mit dem Amid, die mit "#" markierte Bindung mit dem Rest Z verknüpft ist.
M steht besonders bevorzugt für einen Cyclus ausgewählt aus M-1, M-2, M-3, M-4, M-5, M-6, M-9, M-10 und M-11.
M steht ganz besonders bevorzugt für einen Cyclus ausgewählt aus M-1, M-2, M-5, M-6, M-9, M-10 und M-11.
M steht insbesondere bevorzugt für den Cyclus M-1.
M steht außerdem insbesondere bevorzugt für den Heterocyclus M-2.
M steht außerdem insbesondere bevorzugt für den Heterocyclus M-5.
M steht außerdem insbesondere bevorzugt für den Heterocyclus M-6.
M steht außerdem insbesondere bevorzugt für den Heterocyclus M-9.
M steht außerdem insbesondere bevorzugt für den Heterocyclus M-10.
M steht außerdem insbesondere bevorzugt für den Heterocyclus M-11.
R⁷ steht bevorzugt für Wasserstoff.
R⁷ steht für den Fall, dass M für M-1 steht, außerdem bevorzugt für Fluor, wobei Fluor besonders bevorzugt in 4-, 5- oder 6-Position, ganz besonders bevorzugt in 4- oder 6-Position, insbesondere in 4-Position des Anilidrestes steht [vgl. oben Formel (I)].
R⁷ steht für den Fall, dass M für M-1 steht, außerdem bevorzugt für Chlor, wobei Chlor besonders bevorzugt in 5-Position des Anilidrestes steht [vgl. oben Formel (I)].
R⁷ steht für den Fall, dass M für M-1 steht, außerdem bevorzugt für Methyl, wobei Methyl besonders bevorzugt in 3-Position des Anilidrestes steht [vgl. oben Formel (I)].
- R⁷: steht für den Fall, dass M für M-1 steht, außerdem bevorzugt für Trifluormethyl, wobei Trifluormethyl besonders bevorzugt in 4- oder 5-Position des Anilidrestes steht [vgl. oben Formel (I)].
- R⁷: steht für den Fall, dass M für M-2, M-3 oder M-4 steht, außerdem bevorzugt für Chlor, wobei Chlor besonders bevorzugt in 5-Position (M-2, M-3) oder in 3-Position (M-4) steht.
- R⁷: steht für den Fall, dass M für M-2, M-3 oder M-4 steht, außerdem bevorzugt für Methyl, wobei Methyl besonders bevorzugt in 5-Position (M-2, M-3) oder in 3-Position (M-4) steht.
- R⁷: steht für den Fall, dass M für M-5, M-6, M-7 oder M-8 steht, außerdem bevorzugt für Fluor, wobei Fluor besonders bevorzugt in 6-Position (M-5, M-6) oder in 3-Position (M-7, M-8) steht.
- R⁷: steht für den Fall, dass M für M-5, M-6, M-7 oder M-8 steht, außerdem bevorzugt für Chlor, wobei Chlor besonders bevorzugt in 6-Position (M-5, M-6) oder in 3-Position (M-7, M-8) steht.
- R⁷: steht für den Fall, dass M für M-5, M-6, M-7 oder M-8 steht, außerdem bevorzugt für Methyl, wobei Methyl besonders bevorzugt in 4-Position (M-5) oder in 3-Position (M-6, M-7, M-8) steht.
- R⁷: steht für den Fall, dass M für M-9 steht, außerdem bevorzugt für Methyl, wobei Methyl besonders bevorzugt in 3-Position steht.
- R⁷: steht für den Fall, dass M für M-9 steht, außerdem bevorzugt für Trifluormethyl, wobei Trifluormethyl besonders bevorzugt in 3-Position steht.
- R⁷: steht für den Fall, dass M für M-12 steht, außerdem bevorzugt für Methyl, wobei Methyl besonders bevorzugt in 4-Position steht.
- R⁷: steht für den Fall, dass M für M-12 steht, außerdem bevorzugt für Trifluormethyl, wobei Trifluormethyl besonders bevorzugt in 4-Position steht.
- R⁷: steht für den Fall, dass M für M-13 steht, außerdem bevorzugt für Methyl, wobei Methyl besonders bevorzugt in 3-Position steht.
- R⁷: steht für den Fall, dass M für M-13 steht, außerdem bevorzugt für Trifluormethyl, wobei Trifluormethyl besonders bevorzugt in 3-Position steht.
- R⁷: steht für den Fall, dass M für M-14 steht, außerdem bevorzugt für Methyl, wobei Methyl besonders bevorzugt in 3-Position steht.
- R⁷: steht für den Fall, dass M für M-14 steht, außerdem bevorzugt für Trifluormethyl, wobei Trifluormethyl besonders bevorzugt in 3-Position steht.

- R^{7-A}: steht bevorzugt für Wasserstoff.
- R^{7-A}: steht außerdem bevorzugt für Methyl.
- R^{7-A}: steht außerdem bevorzugt für Trifluormethyl.
- Z: steht bevorzugt für Z¹.
- Z¹: steht bevorzugt für gegebenenfalls einfach bis fünffach, gleich oder verschieden substituiertes Phenyl, wobei die Substituenten jeweils aus der Liste W¹ ausgewählt sind.
- Z¹: steht besonders bevorzugt für einfach substituiertes Phenyl, wobei die Substituenten aus der Liste W¹ ausgewählt sind.
- Z¹: steht auch besonders bevorzugt für zweifach, gleich oder verschieden substituiertes Phenyl, wobei die Substituenten aus der Liste W¹ ausgewählt sind.
- Z¹: steht auch besonders bevorzugt für dreifach, gleich oder verschieden substituiertes Phenyl, wobei die Substituenten aus der Liste W¹ ausgewählt sind.
- Z¹: steht ganz besonders bevorzugt für einfach in 4-Position substituiertes Phenyl, wobei die Substituenten aus der Liste W¹ ausgewählt sind.
- Z¹: steht ganz besonders bevorzugt für zweifach, gleich oder verschieden in 3,4-Position substituiertes Phenyl, wobei die Substituenten aus der Liste W¹ ausgewählt sind.
- Z¹: steht ganz besonders bevorzugt für zweifach, gleich oder verschieden in 2,3-Position substituiertes Phenyl, wobei die Substituenten aus der Liste W¹ ausgewählt sind.
- Z¹: steht ganz besonders bevorzugt für zweifach, gleich oder verschieden in 2,4-Position substituiertes Phenyl, wobei die Substituenten aus der Liste W¹ ausgewählt sind.
- Z¹: steht ganz besonders bevorzugt für zweifach, gleich oder verschieden in 3,5-Position substituiertes Phenyl, wobei die Substituenten aus der Liste W¹ ausgewählt sind.
- Z¹: steht ganz besonders bevorzugt für dreifach, gleich oder verschieden in 2,4,6-Position substituiertes Phenyl, wobei die Substituenten aus der Liste W¹ ausgewählt sind.
- W¹: steht für Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocar-bonyl, Dialkylaminocarbonyloxy mit 1 bis 6 Kohlenstoffatomen in den jeweiligen Kohlenwasserstoffketten, Alkenylcarbonyl oder Alkinylcarbonyl, mit 2 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;

oder die Gruppierung -C(Q¹)=N-Q², worin
Q¹ für Wasserstoff, Hydroxy oder Alkyl mit 1 bis 4 Kohlenstoffatomen, Halogenalkyl mit 1 bis 4 Kohlenstoffatomen und 1 bis 9 Fluor-, Chlor- und/oder Bromatomen oder Cycloalkyl mit 1 bis 6 Kohlenstoffatomen steht und
Q² für Hydroxy, Amino, Methylamino, Phenyl, Benzyl oder für jeweils gegebenenfalls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/ oder geradkettiges oder verzweigtes Alkyl oder Alkoxy mit 1 bis 4 Kohlenstoffatomen substituiertes Phenyl, Phenoxy, Phenyllthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen.
- W¹: steht bevorzugt für Fluor, Chlor, Brom, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy, Trifluorethoxy, jeweils zweifach verknüpftes Difluormethylendioxy oder Tetrafluorethylendioxy,oder die Gruppierung -C(Q¹)=N-Q², worin
Q¹ für Wasserstoff, Methyl, Ethyl oder Trifluormethyl steht und
Q² für Hydroxy, Methoxy, Ethoxy, Propoxy oder Isopropoxy steht.
- Z: steht auch bevorzugt für Z².
- Z²: steht bevorzugt für jeweils gegebenenfalls einfach bis vierfach, gleich oder verschieden durch Halogen und/oder C₁-C₄-Alkyl substituiertes Cycloalkyl oder Bicycloalkyl mit jeweils 3 bis 10 Kohlenstoffatomen.
- Z²: steht besonders bevorzugt für jeweils gegebenenfalls einfach bis vierfach, gleich oder verschieden durch Chlor und/oder Methyl substituiertes Cyclopropyl, Cyclopentyl, Cyclohexyl, Cycloheptyl, Cyclooctyl, Cyclononyl, Bicyclo[2.2.1]heptyl oder Bicyclo[2.2.2]octyl.
- Z²: steht ganz besonders bevorzugt für durch Chlor und Methyl substituiertes Cyclopropyl.
- Z: steht auch bevorzugt für Z³.
- Z³: steht bevorzugt für unsubstituiertes C₂-C₂₀-Alkyl oder für einfach oder mehrfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₁-C₂₀-Alkyl, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- Z³: steht besonders bevorzugt für unsubstituiertes C₂-C₂₀-Alkyl.
- Z³: steht auch besonders bevorzugt für durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR⁸R⁹R¹⁰, Cyclopropyl, Dichlorcyclopropyl, Cyclobutyl, Cyclopentyl oder Cyclohexyl substituiertes C₁-C₂₀-Alkyl; ganz besonders bevorzugt für durch Fluor, Chlor, Methylthio, Ethylthio, n- oder iso-Propylthio, n-, iso-, sec-, tert-Butylthio, Pentylthio, Hexylthio, Methylsulfonyl, Ethylsulfonyl, n- oder iso-Propylsulfonyl, n-, iso-, sec-, tert-Butylsulfonyl, Methoxy, Ethoxy, n- oder iso-Propoxy, n-, iso-, sec-, tert-Butoxy, Methylamino, Ethylamino, n- oder iso-Propylamino, n-, iso-, sec-, tert-Butylamino, Dimethylamino, Diisopropylamino, Trifluormethylthio, Trifluormethoxy, -SiR⁸R⁹R¹⁰, Cyclopropyl, Dichlorcyclopropyl, Cyclobutyl, Cyclopentyl oder Cyclohexyl substituiertes C₁-C₂₀-Alkyl.
- Z: steht auch bevorzugt für Z⁴.
- Z⁴: steht bevorzugt für jeweils gegebenenfalls einfach oder mehrfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₂₀-Alkenyl oder C₂-C₂₀-Alkinyl, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- Z⁴: steht besonders bevorzugt für jeweils gegebenenfalls durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR⁸R⁹R¹⁰, Cyclopropyl, Dichlorcyclopropyl, Cyclobutyl, Cyclopentyl oder Cyclohexyl substituiertes C₂-C₂₀-Alkenyl oder C₂-C₂₀-Alkinyl.
- Z⁴: steht ganz besonders bevorzugt für C₂-C₂₀-Alkenyl oder C₂-C₂₀-Alkinyl.
- R⁸ und R⁹: stehen unabhängig voneinander bevorzugt für C₁-C₆-Alkyl, C₁-C₆-Alkoxy, C₁-C₃-Alkoxy-C₁-C₃-alkyl oder C₁-C₃-Alkylthio-C₁-C₃-alkyl.
- R⁸ und R⁹: stehen unabhängig voneinander besonders bevorzugt für Methyl, Ethyl, Methoxy, Ethoxy, Methoxymethyl, Ethoxymethyl, Methoxyethyl, Ethoxyethyl, Methylthiomethyl, Ethylthiomethyl, Methylthioethyl oder Ethylthioethyl.
- R⁸ und R⁹: stehen unabhängig voneinander ganz besonders bevorzugt für Methyl, Methoxy, Methoxymethyl oder Methylthiomethyl.
- R⁸ und R⁹: stehen insbesondere bevorzugt jeweils für Methyl.
- R¹⁰: steht bevorzugt für C₁-C₆-Alkyl, C₁-C₆-Alkoxy, C₁-C₃-Alkoxy-C₁-C₃-alkyl, C₁-C₃-Alkylthio-C₁-C₃-alkyl, C₃-C₆-Cycloalkyl, Phenyl oder Benzyl.
- R¹⁰: steht besonders bevorzugt für Methyl, Ethyl, n- oder iso-Propyl, n-, sec-, iso- oder tert-Butyl, Methoxy, Ethoxy, n- oder iso-Propoxy, n-, sec-, iso- oder tert-Butoxy, Methoxymethyl, Ethoxymethyl, Methoxyethyl, Ethoxyethyl, Methylthiomethyl, Ethylthiomethyl, Methylthioethyl, Ethylthioethyl, Cyclopropyl, Phenyl oder Benzyl.
- R¹⁰: steht ganz besonders bevorzugt für Methyl, Ethyl, n- oder iso-Propyl, iso- oder tert-Butyl, Methoxy, iso-Propoxy, iso- oder tert-Butoxy, Methoxymethyl, Methylthiomethyl oder Phenyl.
- R¹⁰: steht insbesondere bevorzugt für Methyl, Ethyl, n- oder iso-Propyl, iso- oder tert-Butyl, Methoxy, iso-Propoxy, iso- oder tert-Butoxy.
- R¹⁰: steht hervorgehoben für Methyl.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Bevorzugt und jeweils als Teilmenge der oben genannten Verbindungen der Formel (I) zu verstehen sind folgende Gruppen von neuen Carboxamiden:
Gruppe 1: 2-Halogenfuryl/thienyl-3-carboxamide der Formel **Fehler! Verweisquelle konnte nicht gefunden werden.** 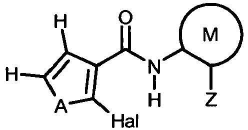 in welcher A, Hal, R, M und Z die oben angegebenen Bedeutungen haben.
Gruppe 3: 2-Halogenfuryl/thienyl-3-carboxamide der Formel (I-d) 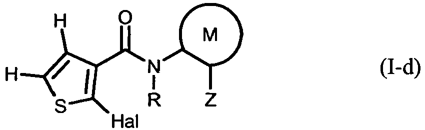 in welcher Hal, R, M und Z die oben angegebenen Bedeutungen haben.
Gruppe 4: 2-Halogenfuryl/thienyl-3-carboxamide der Formel (I-e) 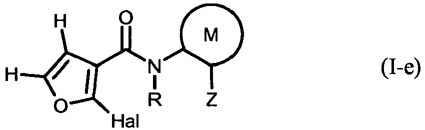 in welcher Hal, R, M und Z die oben angegebenen Bedeutungen haben.
Gruppe 5: 2-Halogenfuryl/thienyl-3-carboxamide der Formel (I-f) 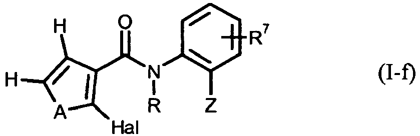 in welcher A, Hal, R, R⁷ und Z die oben angegebenen Bedeutungen haben.
   Bevorzugt sind Verbindungen der Formel (I-f), in welcher A für S (Schwefel) steht.
   Bevorzugt sind außerdem Verbindungen der Formel (I-f), in welcher A für O (Sauerstoff) steht.
Gruppe 6: 2-Halogenfuryl/thienyl-3-carboxamide der Formel (I-g) 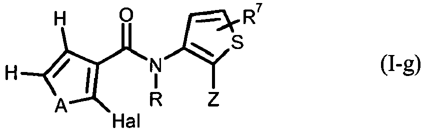 in welcher A, Hal, R, R⁷ und Z die oben angegebenen Bedeutungen haben.
   Bevorzugt sind Verbindungen der Formel (I-g), in welcher A für S (Schwefel) steht.
   Bevorzugt sind außerdem Verbindungen der Formel (I-g), in welcher A für O (Sauerstoff) steht.
Gruppe 7: 2-Halogenfuryl/thienyl-3-carboxamide der Formel (I-h) 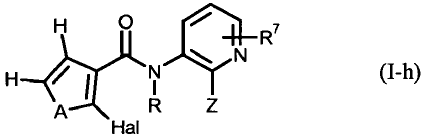 in welcher A, Hal, R, R⁷ und Z die oben angegebenen Bedeutungen haben.
   Bevorzugt sind Verbindungen der Formel (I-h), in welcher A für S (Schwefel) steht.
   Bevorzugt sind außerdem Verbindungen der Formel (I-h), in welcher A für O (Sauerstoff) steht.
Gruppe 8: 2-Halogenfuryl/thienyl-3-carboxamide der Formel (I-i) 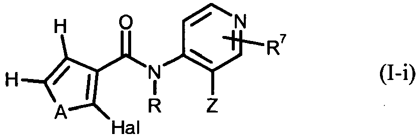 in welcher A, Hal, R, R⁷ und Z die oben angegebenen Bedeutungen haben.
   Bevorzugt sind Verbindungen der Formel (I-i), in welcher A für S (Schwefel) steht.
   Bevorzugt sind außerdem Verbindungen der Formel (I-i), in welcher A für O (Sauerstoff) steht.
Gruppe 9: 2-Halogenfuryl/thienyl-3-carboxamide der Formel (I-j) 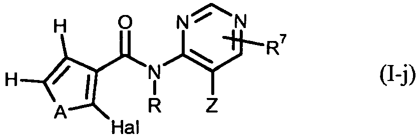 in welcher A, Hal, R, R⁷ und Z die oben angegebenen Bedeutungen haben.
   Bevorzugt sind Verbindungen der Formel (I-j), in welcher A für S (Schwefel) steht.
   Bevorzugt sind außerdem Verbindungen der Formel (I-j), in welcher A für O (Sauerstoff) steht.
Gruppe 10: 2-Halogenfuryl/thienyl-3-carboxamide der Formel (I-k) 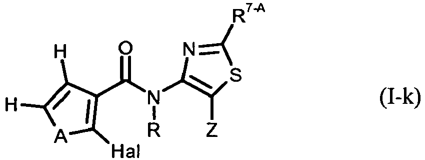 in welcher A, Hal, R, R⁷ und Z die oben angegebenen Bedeutungen haben.
   Bevorzugt sind Verbindungen der Formel (I-k), in welcher A für S (Schwefel) steht.
   Bevorzugt sind außerdem Verbindungen der Formel (I-k), in welcher A für O (Sauerstoff) steht.
Gruppe 11: 2-Halogenfuryl/thienyl-3-carboxamide der Formel (I-1) 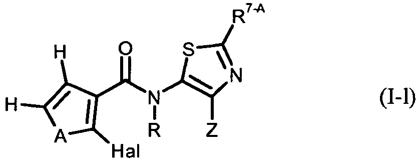 in welcher A, Hal, R, R⁷ und Z die oben angegebenen Bedeutungen haben.
   Bevorzugt sind Verbindungen der Formel (I-1), in welcher A für S (Schwefel) steht.
   Bevorzugt sind außerdem Verbindungen der Formel (I-1), in welcher A für O (Sauerstoff) steht.
Gruppe 12: 2-Halogenfuryl/thienyl-3-carboxamide der Formel (I-m) 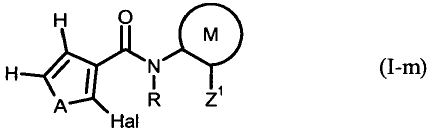 in welcher A, Hal, R, M und Z¹ die oben angegebenen Bedeutungen haben.
   Bevorzugt sind Verbindungen der Formel (I-m), in welcher A für S (Schwefel) steht.
   Bevorzugt sind außerdem Verbindungen der Formel (I-m), in welcher A für O (Sauerstoff) steht.
Gruppe 13: 2-Halogenfuryl/thienyl-3-carboxamide der Formel (I-n) 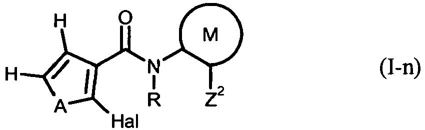 in welcher A, Hal, R, M und Z² die oben angegebenen Bedeutungen haben.
   Bevorzugt sind Verbindungen der Formel (I-n), in welcher A für S (Schwefel) steht.
   Bevorzugt sind außerdem Verbindungen der Formel (I-n), in welcher A für O (Sauerstoff) steht.
Gruppe 14: 2-Halogenfuryl/thienyl-3-carboxamide der Formel (I-o) 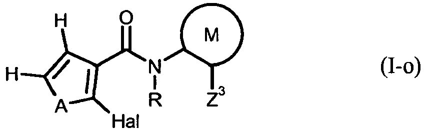 in welcher A, Hal, R, M und Z³ die oben angegebenen Bedeutungen haben.
   Bevorzugt sind Verbindungen der Formel (I-o), in welcher A für S (Schwefel) steht.
   Bevorzugt sind außerdem Verbindungen der Formel (I-o), in welcher A für O (Sauerstoff) steht.
Gruppe 15: 2-Halogenfuryl/thienyl-3-carboxamide der Formel (I-p) 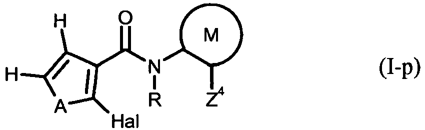 in welcher A, Hal, R, M und Z⁴ die oben angegebenen Bedeutungen haben.
   Bevorzugt sind Verbindungen der Formel (I-p), in welcher A für S (Schwefel) steht.
   Bevorzugt sind außerdem Verbindungen der Formel (I-p), in welcher A für O (Sauerstoff) steht.
Gruppe 16: 2-Halogenfuryl/thienyl-3-carboxamide der Formel (I-q) 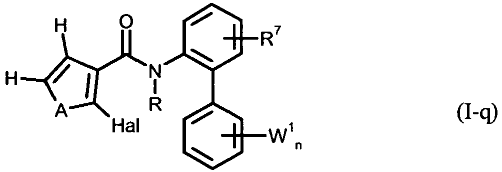 in welcher A, Hal, R, R⁷ und W¹ die oben angegebenen Bedeutungen haben und n für 0, 1, 2, 3, 4 oder 5 steht, wobei die Substituenten W¹ gleich oder verschieden sein können, wenn n für 2, 3, 4 oder 5 steht.
   Bevorzugt sind Verbindungen der Formel (I-q), in welcher A für S (Schwefel) steht.
   Bevorzugt sind außerdem Verbindungen der Formel (I-q), in welcher A für O (Sauerstoff) steht.
Gruppe 17: 2-Halogenfuryl/thienyl-3-carboxamide der Formel (I-r) 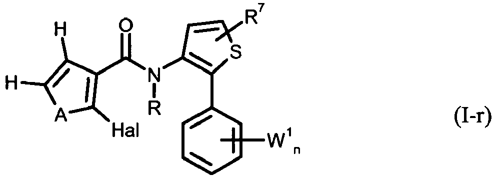 in welcher A, Hal, R, R⁷ und W¹ die oben angegebenen Bedeutungen haben und n für 0, 1, 2, 3, 4 oder 5 steht, wobei die Substituenten W¹ gleich oder verschieden sein können, wenn n für 2,3,4 oder 5 steht.
   Bevorzugt sind Verbindungen der Formel (I-r), in welcher A für S (Schwefel) steht.
   Bevorzugt sind außerdem Verbindungen der Formel (I-r), in welcher A für O (Sauerstoff) steht.
Gruppe 18: 2-Halogenfuryl/thienyl-3-carboxamide der Formel (I-s) 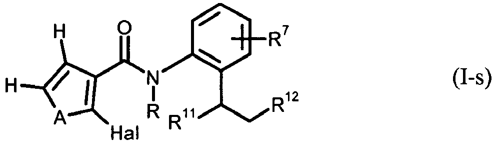 in welcher
   - A, Hal, R und R⁷: die oben angegebenen Bedeutungen haben,
   - R¹¹: für Wasserstoff oder Methyl steht,
   - R¹²: für -SiR⁸R⁹R¹⁰ steht, wobei R⁸, R⁹ und R¹⁰ die oben angegebenen Bedeutungen haben, oder für -CR¹³R¹⁴R¹⁵ steht, wobei R¹³ für Fluor, Chlor, Brom, Iod, C₁-C₄-Alkylthio, C₁-C₄-Alkyl-sulfmyl, C₁-C₄-Alkylsulfonyl, C₁-C₄-Alkoxy, C₁-C₄-Alkylamino, Di(C₁-C₄-alkyl)amino, C₁-C₄-Halogenalkyl, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino oder Halogen-di(C₁-C₄-alkyl)amino steht, und R¹⁴ und R¹⁵ unabhängig voneinander für Wasserstoff, Fluor, Chlor, Brom, Iod, C₁-C₄-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino oder Halogen-di(C₁-C₄-alkyl)-amino stehen.
   Bevorzugt sind Verbindungen der Formel (I-s), in welcher A für S (Schwefel) steht.
   Bevorzugt sind außerdem Verbindungen der Formel (I-s), in welcher A für O (Sauerstoff) steht.
Gruppe 19: 2-Halogenfuryl/thienyl-3-carboxamide der Formel (I-t) 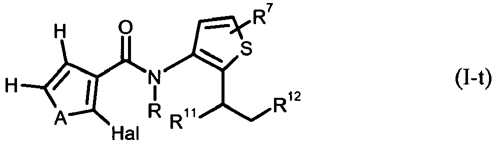 in welcher
   - A, Hal, R und R⁷: die oben angegebenen Bedeutungen haben,
   - R¹¹: für Wasserstoff oder Methyl steht,
   - R¹²: für -SiR⁸R⁹R¹⁰ steht, wobei R⁸, R⁹ und R¹⁰ die oben angegebenen Bedeutungen haben, oder für -CR¹³R¹⁴R¹⁵ steht, wobei R¹³ für Fluor, Chlor, Brom, Iod, C₁-C₄-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkyl, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino oder Halogen-di(C₁-C₄-alkyl)amino steht, und R¹⁴ und R¹⁵ unabhängig voneinander für Wasserstoff, Fluor, Chlor, Brom, Iod, C₁-C₄-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino oder Halogen-di(C₁-C₄-alkyl)amino stehen.

Bevorzugt sind Verbindungen der Formel (I-t), in welcher A für S (Schwefel) steht.

Bevorzugt sind außerdem Verbindungen der Formel (I-t), in welcher A für O (Sauerstoff) steht. Hervorgehoben sind Verbindungen der Formel (I) (und ebenso der Gruppen 1 bis 19), in welcher R für Wasserstoff steht.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein. Die Definition C₁-C₂₀-Alkyl umfasst den größten hierin definierten Bereich für einen Alkylrest. Im Einzelnen umfasst diese Definition die Bedeutungen Methyl, Ethyl, n-, iso-Propyl, n-, iso-, sec-, tert-Butyl, sowie jeweils alle isomeren Pentyle, Hexyle, Heptyle, Octyle, Nonyle, Decyle, Undecyle, Dodecyle, Tridecyle, Tetradecyle, Pentadecyle, Hexadecyle, Heptadecyle, Octadecyle, Nonadecyle und Eicosyle. Ein bevorzugter Bereich ist C₂-C₁₂-Alkyl wie Ethyl und geradkettiges oder verzweigtes Propyl, Butyl, Pentyl, Hexyl, Heptyl, Octyl, Nonyl, Decyl, Undecyl und Dodecyl, besonders geradkettiges oder verzweigtes C₃-C₁₀-Alkyl wie Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl, insbesondere Propyl, 1-Methylethyl, Butyl, 1-Methylbutyl, 2-Methylbutyl, 3-Methylbutyl, 1,1-Dimethylethyl, 1,2-Dimethylbutyl, 1,3-Dimethylbutyl, Pentyl, 1-Methylbutyl, 1-Ethylpropyl, Hexyl, 3-Methylpentyl, Heptyl, 1-Methylhexyl, 1-Ethyl-3-methylbutyl, 1-Methylheptyl, 1,2-Dimethylhexyl, 1,3-Dimethyloctyl, 4-Methyloctyl, 1,2,2,3-Tetramethylbutyl, 1,3,3-Trimethylbutyl, 1,2,3-Trimethylbutyl, 1,3-Dimethylpentyl, 1,3-Dimethylhexyl, 5-Methyl-3-hexyl, 2-Methyl-4-heptyl, 2,6-Dimethyl-4-heptyl und 1-Methyl-2-cyclopropylethyl.

Durch Halogen substituiertes Alkyl steht beispielsweise für Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlor-1-methylbutyl, 2-Chlor-1-methylbutyl, 1-Chlorbutyl, 3,3-Dichlor-1-methylbutyl, 3-Chlor-1-methylbutyl, 1-Methyl-3-trifluormethylbutyl, 3-Methyl-1-trifluormethylbutyl.

Der Substituent -SiR⁸R⁹R¹⁰ steht bevorzugt für folgende Reste: SiMe₃, SiMe₂Et, SiMe₂CHMe₂, SiMe₂CH₂CHMe₂, SiMe₂CH₂CMe₃, SiMe₂OCHMe₂, SiMe₂OCH₂CHMe₂, SiMe₂OMe, SiMe₂CMe₃, SiMe₂CH₂CH₂Me.

Die Definition C₂-C₂₀-Alkenyl umfasst den größten hierin definierten Bereich für einen Alkenylrest. Im Einzelnen umfasst diese Definition die Bedeutungen Ethenyl, n-, iso-Propenyl, n-, iso-, sec-, tert-Butenyl, sowie jeweils alle isomeren Pentenyle, Hexenyle, Heptenyle, Octenyle, Nonenyle, Decenyle, Undecenyle, Dodecenyle, Tridecenyle, Tetradecenyle, Pentadecenyle, Hexadecenyle, Heptadecenyle, Octadecenyle, Nonadecenyle und Eicosenyle, 1-Methyl-1-propenyl, 1-Ethyl-1-butenyl, 2,4-Dimethyl-1-pentenyl, 2,4-Dimethyl-2-pentenyl.

Die Definition C₂-C₂₀-Alkinyl umfasst den größten hierin definierten Bereich für einen Alkinylrest. Im Einzelnen umfasst diese Definition die Bedeutungen Ethinyl, n-, iso-Propinyl, n-, iso-, sec-, tert-Butinyl, sowie jeweils alle isomeren Pentinyle, Hexinyle, Heptinyle, Octinyle, Noninyle, Decinyle, Undecinyle, Dodecinyle, Tridecinyle, Tetradecinyle, Pentadecinyle, Hexadecinyle, Heptadecinyle, Octadecinyle, Nonadecinyle und Eicosinyle.

Gegebenenfalls substituierte Reste können einfach oder mehrfach substituiert sein, wobei bei Mehrfachsubstitutionen die Substituenten gleich oder verschieden sein können. So schließt die Definition Dialkylamino auch eine unsymmetrisch durch Alkyl substituierte Aminogruppe wie z.B. Methyl-ethylamino ein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend. Insbesondere können die in den Gruppen 1 bis 18 genannten Verbindungen sowohl mit den allgemeinen wie auch mit bevorzugten, besonders bevorzugten usw. Bedeutungen kombiniert werden, wobei auch hier jeweils alle Kombinationen zwischen den Vorzugsbereichen möglich sind. Beispielsweise können die Reste Hal und Z in der Formel (I-d) die allgemeinen Bedeutungen haben, während R die bevorzugten und M die besonders bevorzugten Bedeutungen hat.

### Erläuterungen der Verfahren und Zwischenprodukte:

### Verfahren (a)

Verwendet man 2-Iodthiophen-3-carbonsäurechlorid und 3',4'-Dichlor-1,1'-biphenyl-2-amin als Ausgangsstoffe, so kann der Verlauf des erfindungsgemäßen Verfahrens (a) durch das folgende Formelschema veranschaulicht werden.

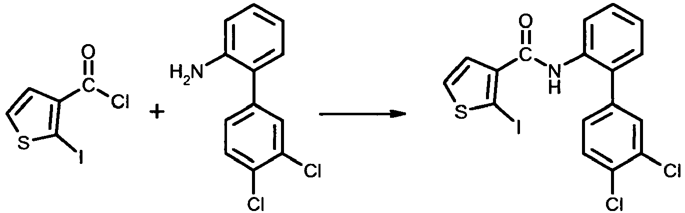

Die zur Durchführung des erfindungsgemäßen Verfahrens (a) als Ausgangsstoffe benötigten Carbonsäure-Derivate sind durch die Formel (II) allgemein definiert. In dieser Formel (II) haben A und Hal bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt diese Reste angegeben wurden. X¹ steht bevorzugt für Chlor oder Hydroxy.

Verwendet man 2-Iodthiophen-3-carbonsäure und 3',4'-Dichlor-1,1'-biphenyl-2-amin als Ausgangsstoffe in Gegenwart von Kupplungsreagenzien, wie z.B. Dicyclohexylcarbodiimid, oder erzeugt das Säurehalogenid *in situ,* z.B. mit PyBrop (Bromo-tris-pyrrolidino-phosphonium hexafluorophosphat) oder Oxalylchlorid, so kann der Verlauf des erfindungsgemäßen Verfahrens (a) durch das folgende Formelschema veranschaulicht werden.

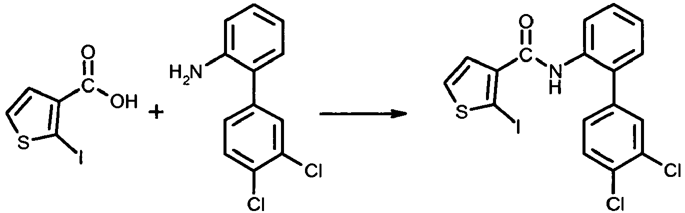

Die Carbonsäure-Derivate der Formel (II) sind bekannt und/oder können nach bekannten Verfahren hergestellt werden (Heterocycles 1993, 36, 1867).

Die zur Durchführung des erfindungsgemäßen Verfahrens (a) weiterhin als Ausgangsstoffe benötigten Anilin-Derivate sind durch die Formel (III) allgemein definiert. In dieser Formel (III) haben R, M und Z bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Ausgangsstoffe der Formel (III) sind größtenteils bekannt und/oder können nach bekannten Verfahren hergestellt werden (vgl. z.B. Bull. Korean Chem. Soc. 2000, 21, 165-166; Chem. Pharm. Bull. 1992, 40, 240-244; Heterocycles 1989, 29, 1013-1016; J. Med. Chem. 1996, 39, 892-903; Synthesis 1995, 713-16; Synth. Commun. 1994, 24, 267-272; Synthesis 1994, 142-144; DE-A 27 27 416; EP-A 0 824 099; EP-A 0 737 682, WO 93/11117, WO 03/080628; EP-A 0 545 099, EP-A 0 589 301, EP-A 0 589 313 und WO 02/38542).

Es ist auch möglich, zunächst Anilin-Derivate der Formel (III-a) 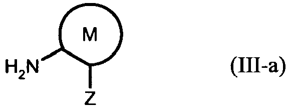 in welcher M und Z die oben angegebenen Bedeutungen haben, herzustellen und diese gegebenenfalls anschließend mit Halogeniden der Formel **Fehler! Verweisquelle konnte nicht gefunden werden.**

R^{a}-X⁶ **Fehler! Verweisquelle konnte nicht gefunden werden.**

in welcher R^{a} und X⁶ die oben angegebenen Bedeutungen haben, in Gegenwart einer Base und in Gegenwart eines Verdünnungsmittels umzusetzen. [Die Reaktionsbedingungen des erfindungsgemäßen Verfahrens (i) gelten entsprechend.]

Anilin-Derivate der Formel (III-b) 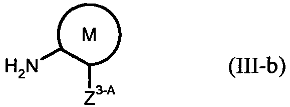 in welcher
- M: die oben angegebenen Bedeutungen hat und
- Z^{3-A}: für einfach durch Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino substituiertes C₁-C₂₀-Alkyl steht,
werden beispielsweise gemäß folgenden Schema 1 erhalten:

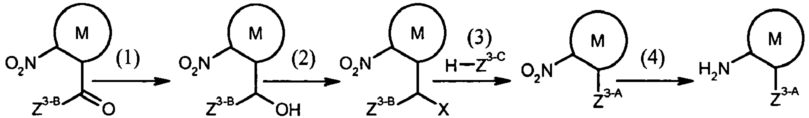

In diesem Schema 1 hat Z^{3-A} die oben angegebenen Bedeutungen. Z^{3-B} steht für unsubstituiertes C₂-C₂₀-Alkyl, Z³-C für Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino oder Halogen-dialkylamino. X steht für Chlor, Brom oder Iod. Die für Z³ angegebenen Vorzugsbereiche gelten für die hier zur Anwendung kommenden Bedeutungen entsprechend.

Anilin-Derivate der Formel (III-c) 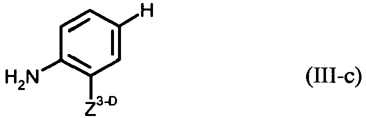 in welcher Z^{3-D} für einfach oder mehrfach durch Halogen substituiertes C₁-C₂₀-Alkyl steht, werden beispielsweise gemäß folgenden Schema 2 erhalten:

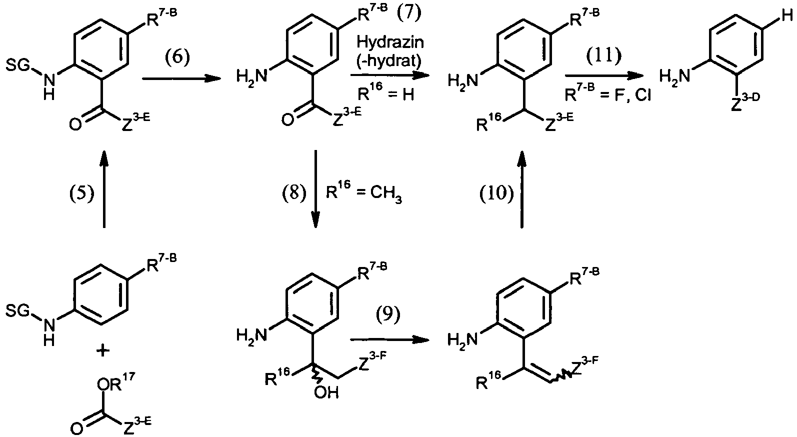

In diesem Schema 2 hat Z^{3-D} die oben angegebenen Bedeutungen. Z^{3-E} steht für einfach oder mehrfach durch Halogen substituiertes C₁-C₁₉-Alkyl. Z^{3-F} steht für einfach oder mehrfach durch Halogen substituiertes C₁-C₁₇-Alkyl. R^{7-B} steht für Fluor, Chlor, Methyl, iso-Propyl, Methylthio oder Trifluormethyl. R¹⁶ steht für Wasserstoff oder Methyl. SG steht für eine Schutzgruppe, bevorzugt Piv (tert-Butylcarbonyl), Boc (tert-Butoxycarbonyl-), Cbz (Benzyloxycarbonyl-), Trifluoracetyl-, Fmoc (9-Fluorenylmethoxycarbonyl-) oder Troc (2,2,2-Trichlorethoxycarbonyl-). R¹⁷ steht für C₁-C₄-Alkyl, bevorzugt Methyl oder Ethyl.

Die für Z³ und R⁷ angegebenen Vorzugsbereiche gelten für die hier zur Anwendung kommenden Bedeutungen entsprechend.

### Verfahren (b)

Verwendet man N-(2-Bromphenyl)-2-iodthiophen-3-carboxamid und 3,4-Dichlorphenylboronsäure als Ausgangsstoffe sowie einen Katalysator, so kann der Verlauf des erfindungsgemäßen Verfahrens (b) durch das folgende Formelschema veranschaulicht werden.

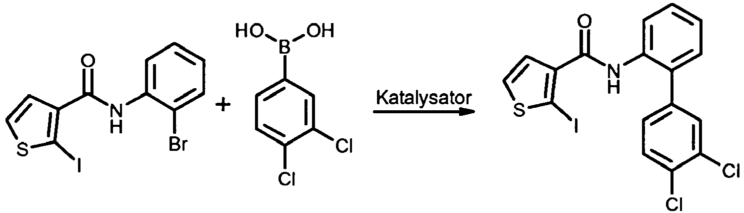

Die zur Durchführung des erfindungsgemäßen Verfahrens (b) als Ausgangsstoffe benötigten Halogencarboxamide sind durch die Formel (IV) allgemein definiert. In dieser Formel (IV)haben A, Hal, R und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. X² steht für Brom oder Iod.

Die Halogencarboxamide der Formel (IV) sind noch nicht bekannt. Sie sind als neue chemische Verbindungen ebenfalls Gegenstand der vorliegenden Anmeldung. Sie werden erhalten, indem man
j) Carbonsäure-Derivate der Formel (II) 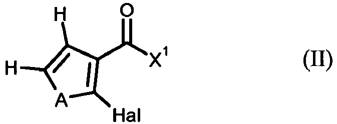 in welcher A, Hal und X¹ die oben angegebenen Bedeutungen haben,
   mit Halogenanilinen der Formel (XTV), 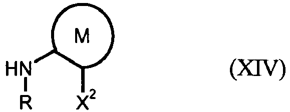 in welcher R, M und X² die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

Die zur Durchführung des erfindungsgemäßen Verfahrens (j) als Ausgangsstoffe benötigten Carbonsäure-Derivate der Formel (II) sind bereits im Zusammenhang mit dem erfindungsgemäßen Verfahren (a) beschrieben worden.

Die zur Durchführung des erfindungsgemäßen Verfahrens (j) weiterhin als Ausgangsstoffe benötigten Halogenaniline sind durch die Formel (XIV) allgemein definiert. In dieser Formel (XIV) haben R, M und X² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) bzw. der Vorprodukte der Formel (III) als bevorzugt, besonders bevorzugt bzw. ganz besonders bevorzugt für diese Reste angegeben wurden.

Die Halogenaniline der Formel (XIV) sind handelsübliche Synthesechemikalien oder können nach bekannten Verfahren erhalten werden.

Es ist auch möglich, zunächst Halogenaniline der Formel (XIV-a) 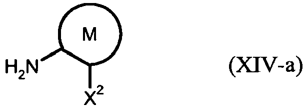 in welcher M und X² die oben angegebenen Bedeutungen haben, herzustellen und diese gegebenenfalls anschließend mit Halogeniden der Formel **Fehler! Verweisquelle konnte nicht gefunden werden.**

R^{a}-X⁶ **Fehler! Verweisquelle konnte nicht gefunden werden.**

in welcher R^{a} und X⁶ die oben angegebenen Bedeutungen haben, in Gegenwart einer Base und in Gegenwart eines Verdünnungsmittels umzusetzen. [Die Reaktionsbedingungen des erfindungsgemäßen Verfahrens (i) gelten entsprechend.]

Die zur Durchführung des erfindungsgemäßen Verfahrens (b) weiterhin als Ausgangsstoffe benötigten Boronsäure-Derivate sind durch die Formel (V) allgemein definiert. In dieser Formel (V) hat Z¹ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für Z¹ angegeben wurden. G¹ und G² stehen jeweils für Wasserstoff oder zusammen für Tetramethylethylen.

Die Boronsäure-Derivate der Formel (V) sind bekannt und/oder lassen sich nach bekannten Verfahren herstellen (vgl. z.B. WO 01/90084 und US 5,633,218).

### Verfahren c)

Verwendet man (2-{[(2-Iod-3-thienyl)carbonyl]amino}phenyl)boronsäure und 4-Brom-1,2-dichlorbenzol als Ausgangsstoffe sowie einen Katalysator, so kann der Verlauf des erfindungsgemäßen Verfahrens (c) durch das folgende Formelschema veranschaulicht werden.

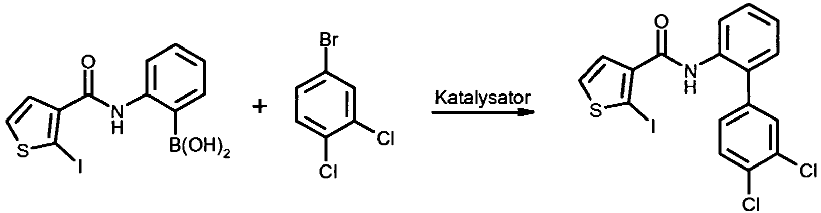

Die zur Durchführung des erfindungsgemäßen Verfahrens (c) als Ausgangsstoffe benötigten Boronsäure-Derivate sind durch die Formel (VI) allgemein definiert. In dieser Formel (VI) haben A, Hal, R und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. G³ und G⁴ stehen jeweils für Wasserstoff oder zusammen für Tetramethylethylen.

Die Boronsäure-Derivate der Formel (VI) sind noch nicht bekannt. Sie sind neue chemische Verbindungen und ebenfalls Gegenstand der vorliegenden Anmeldung. Sie werden erhalten, indem man
k) ein Carbonsäure-Derivat der Formel (II) 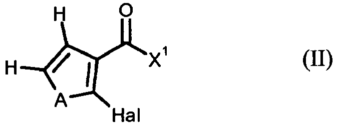 in welcher A, Hal und X² die oben angegebenen Bedeutungen haben,
   mit einem Anilinboronsäure-Derivat der Formel (XV) 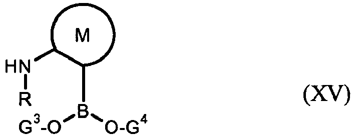 in welcher R, M, G³ und G⁴ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

Die zur Durchführung des erfindungsgemäßen Verfahrens (k) als Ausgangsstoffe benötigten Carbonsäure-Derivate der Formel (II) sind bereits im Zusammenhang mit dem erfindungsgemäßen Verfahren (a) beschrieben worden.

Die weiterhin zur Durchführung des erfindungsgemäßen Verfahrens (k) als Ausgangsstoffe benötigten Anilinboronsäure-Derivate sind durch die Formel (XV) allgemein definiert. In dieser Formel (XV) haben R und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden. G³ und G⁴ stehen jeweils für Wasserstoff oder gemeinsam für Tetramethylethylen.

Die Anilinboronsäure-Derivate der Formel (XV) sind bekannte Synthesechemikalien oder können nach bekannten Verfahren erhalten werden.

Es ist auch möglich, zunächst Anilinboronsäure-Derivate der Formel (XV-a) 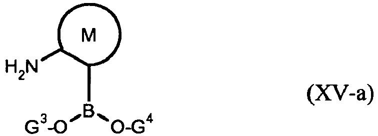 in welcher M, G³ und G⁴ die oben angegebenen Bedeutungen haben, herzustellen und diese gegebenenfalls anschließend mit Halogeniden der Formel **Fehler! Verweisquelle konnte nicht gefunden werden.**

R^{a}-X⁶ **Fehler! Verweisquelle konnte nicht gefunden werden.**

in welcher R^{a} und X⁶ die oben angegebenen Bedeutungen haben, in Gegenwart einer Base und in Gegenwart eines Verdünnungsmittels umzusetzen. [Die Reaktionsbedingungen des erfindungsgemäßen Verfahrens (i) gelten entsprechend.]

Die zur Durchführung des erfindungsgemäßen Verfahrens (c) weiterhin als Ausgangsstoffe benötigten Phenyl-Derivate sind durch die Formel (VII) allgemein definiert. In dieser Formel (VII) hat Z¹ bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für Z¹ angegeben wurden. X³ steht für Chlor, Brom, Iod oder Trifluormethylsulfonat.

Die Phenyl-Derivate der Formel (VII) sind bekannte Synthesechemikalien.

### Verfahren d)

Verwendet man N-(2-Bromphenyl)-2-iodthiophen-3-carboxamid und 4-Brom-1,2-dichlorbenzol als Ausgangsstoffe sowie einen Katalysator und 4,4,4',4',5,5,5',5'-Octamethyl-2,2'-bis-1,3,2-dioxaborolan, so kann der Verlauf des erfindungsgemäßen Verfahrens (d) durch das folgende Formelschema veranschaulicht werden.

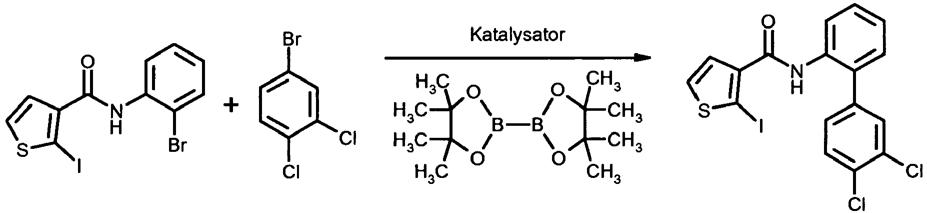

Die zur Durchführung des erfindungsgemäßen Verfahrens (d) als Ausgangsstoffe benötigten Halogencarboxamide der Formel (IV), sowie die Phenyl-Derivate der Formel (VII) sind bereits im Zusammenhang mit den erfindungsgemäßen Verfahren (b) und (c) beschrieben worden.

Das weiterhin zur Durchführung des erfindungsgemäßen Verfahrens (d) benötigte 4,4,4',4',5,5,5',5'-Octamethyl-2,2'-bis-1,3,2-dioxaborolan ist eine handelsübliche Synthesechemikalie.

### Verfahren e)

Hydriert man beispielsweise N-[2-(1,3-Dimethylbut-1-en-1-yl)phenyl]-2-iodthiophen-3-carboxamid, so kann der Verlauf des erfindungsgemäßen Verfahrens (e) durch das folgende Formelschema veranschaulicht werden.

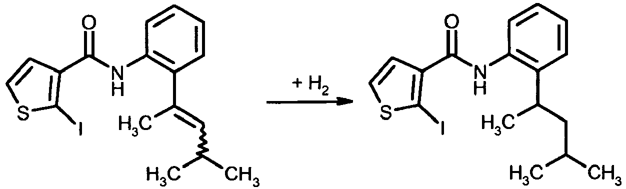

Die zur Durchführung des erfindungsgemäßen Verfahrens (e) als Ausgangsstoffe benötigten 2-Halogenfuryl/thienyl-3-carboxamide sind durch die Formel (I-a) allgemein definiert. In dieser Formel (I-a) haben A, Hal, R und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.
- X⁴: steht bevorzugt für jeweils gegebenenfalls einfach oder mehrfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann.
- X⁴: steht besonders bevorzugt für jeweils gegebenenfalls durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di-(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR⁸R⁹R¹⁰, Cyclopropyl, Dichlorcyclopropyl, Cyclobutyl, Cyclopentyl oder Cyclohexyl substituiertes C₂-C₂₀-Alkenyl oder C₂-C₂₀-Alkinyl.
- X⁴: steht ganz besonders bevorzugt für C₂-C₂₀-Alkenyl oder C₂-C₂₀-Alkinyl.

Die Verbindungen der Formel (I-a) sind erfindungsgemäße Verbindungen und können nach den erfindungsgemäßen Verfahren (a), (f), (g) oder (h) hergestellt werden.

### Verfahren (f)

Dehydratisiert man beispielsweise N-[2-(1-Hydroxy-1,3-dimethylbutyl)phenyl]-2-iodthiophen-3-carboxamid, so kann der Verlauf des erfindungsgemäßen Verfahrens (f) durch das folgende Formelschema veranschaulicht werden.

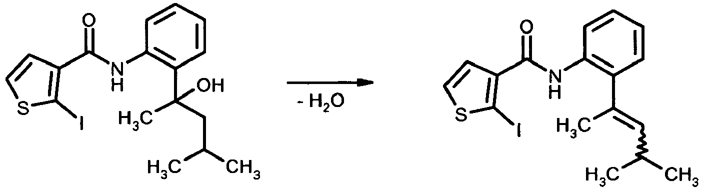

Die zur Durchführung des erfindungsgemäßen Verfahrens (f) als Ausgangsstoffe benötigten Hydroxyalkylcarboxamide sind durch die Formel (VIII) allgemein definiert. In dieser Formel (VIII) haben A, Hal, R und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.
- X⁵: steht bevorzugt für gegebenenfalls zusätzlich einfach bis vierfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkyllhio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₁₂- Hydroxyalkyl, wobei der Cycloalkylteil seinerseits gegebenenfalls durch Halogen und/oder C₁-C₄-Alkyl substituiert sein kann.
- X⁵: steht besonders bevorzugt für jeweils gegebenenfalls einfach bis vierfach, gleich oder verschieden durch Fluor, Chlor, Brom, Iod, C₁-C₆-Alkylthio, C₁-C₄-Alkylsulfinyl, C₁-C₄-Alkyl-sulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR⁸R⁹R¹⁰, Cyclopropyl, Dichlorcyclopropyl, Cyclobutyl, Cyclopentyl oder Cyclohexyl substituiertes, jeweils geradkettiges oder verzweigtes, jeweils an beliebiger Stelle verknüpftes Hydroxyethyl, Hydroxypropyl, Hydroxybutyl, Hydroxypentyl, Hydroxyhexyl, Hydroxyheptyl, Hydroxyoctyl, Hydroxynonyl oder Hydroxydecyl.

Die Verbindungen der Formel (VIII) sind noch nicht bekannt und als neue Verbindungen ebenfalls Gegenstand der vorliegenden Anmeldung.

Es wurde auch gefunden, dass die Hydroxyalkylpyrazolylcarboxamide der Formel (VIII) sehr gute mikrobizide Eigenschaften besitzen und zur Bekämpfung unerwünschter Mikroorganismen sowohl im Pflanzenschutz als auch im Materialschutz eingesetzt werden können.

Die Hydroxyalkylpyrazolylcarboxamide der Formel (VIII) werden erhalten, indem man
l) Carbonsäure-Derivate der Formel (II) 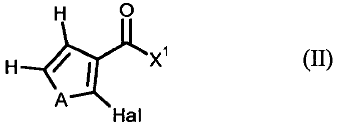 in welcher A, Hal und X¹ die oben angegebenen Bedeutungen haben,
   mit Hydroxyalkylanilin-Derivaten der Formel (XVI) 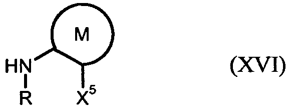 in welcher R, M und X⁵ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

Die zur Durchführung des erfindungsgemäßen Verfahrens (l) als Ausgangsstoffe benötigten Carbonsäure-Derivate der Formel (II) sind bereits im Zusammenhang mit dem erfindungsgemäßen Verfahren (a) beschrieben worden.

Die zur Durchführung des erfindungsgemäßen Verfahrens (l) als Ausgangsstoffe weiterhin benötigten Hydroxyalkylanilin-Derivate sind durch die Formel (XVI) allgemein definiert. In dieser Formel (XVI) haben R, M und X⁵ bevorzugt, besonders bevorzugt bzw. ganz besonders bevorzugt diejenigen Bedeutungen, die bereits im Zusammenhang mit der Beschreibung der erfindungsgemäßen Verbindungen der Formeln (I) bzw. (VIII) als bevorzugt, besonders bevorzugt bzw. ganz besonders bevorzugt für diese Reste angegeben wurden.

Die Hydroxyalkylanilin-Derivate der Formel (XVI) sind bekannt und/oder können nach bekannten Methoden erhalten werden (vgl. z.B. US 3,917,592 oder EP-A 0 824 099).

Es ist auch möglich, zunächst Hydroxyalkylanilin-Derivate der Formel (XVI-a) 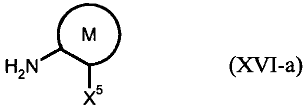 in welcher M, und X⁵ die oben angegebenen Bedeutungen haben, herzustellen und diese gegebenenfalls anschließend mit Halogeniden der Formel **Fehler! Verweisquelle konnte nicht gefunden werden.**

R^{a}-X⁶ **Fehler! Verweisquelle konnte nicht gefunden werden.**

in welcher R^{a} und X⁶ die oben angegebenen Bedeutungen haben, in Gegenwart einer Base und in Gegenwart eines Verdünnungsmittels umzusetzen. [Die Reaktionsbedingungen des erfindungsgemäßen Verfahrens (i) gelten entsprechend.]

### Verfahren (g)

Verwendet man beispielsweise N-(2-Bromphenyl)-2-iodthiophen-3-carboxamid und 3-methylbut-1-in als Ausgangsstoffe sowie einen Katalysator, so kann der Verlauf des erfindungsgemäßen Verfahrens (g) durch das folgende Formelschema veranschaulicht werden.

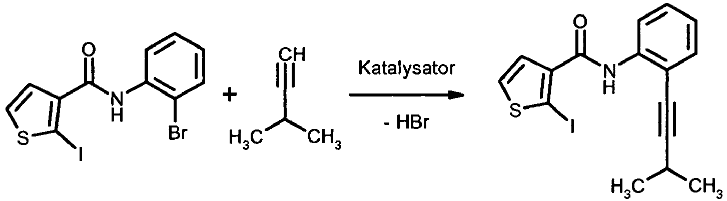

Die zur Durchführung des erfindungsgemäßen Verfahrens (g) als Ausgangsstoffe benötigten Halogencarboxamide der Formel (IV) sind bereits im Zusammenhang mit dem erfindungsgemäßen Verfahrens (c) beschrieben worden.

Die zur Durchführung des erfindungsgemäßen Verfahrens (g) weiterhin als Ausgangsstoffe benötigten Alkine sind durch die Formel (IX) allgemein definiert.
- G⁵: steht bevorzugt für gegebenenfalls einfach bis vierfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- G⁵: steht besonders bevorzugt für jeweils gegebenenfalls einfach bis vierfach, gleich oder verschieden durch Fluor, Chlor, Brom, Iod, C₁-C₆-Alkylthio, C₁-C₄-Alkylsulfinyl, C₁-C₄-Alkyl-sulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR⁸R⁹R¹⁰, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Alkine der Formel (IX) sind bekannte Synthesechemikalien.

Die zur Durchführung des erfindungsgemäßen Verfahrens (g) weiterhin alternativ als Ausgangsstoffe benötigten Alkene sind durch die Formel (X) allgemein definiert.
- G⁶, G⁷ und G⁸: stehen unabhängig voneinander bevorzugt jeweils für Wasserstoff oder jeweils gegebenenfalls einfach bis vierfach, gleich oder verschieden durch Fluor, Chlor, Brom, Iod, C₁-C₆-Al-kylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)-amino, -SiR⁸R⁹R¹⁰ und/oder C₃-C₆-Cycloalkyl substituiertes Alkyl, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils die Zahl 12 nicht übersteigt.
- G⁶, G⁷ und G⁸: stehen unabhängig voneinander besonders bevorzugt jeweils für Wasserstoff oder gegebenenfalls einfach bis vierfach, gleich oder verschieden durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)-amino mit jeweils 1 bis 9 Fluor-, Chlor- und/oder Bromatomen, -SiR⁸R⁹R¹⁰, Cyclopropyl, Difluorcyclopropyl, Cyclobutyl, Cyclopentyl und/ oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl, wobei die Gesamtzahl der Kohlenstoffatome des offenkettigen Molekülteils die Zahl 12 nicht übersteigt.

Die Alkene der Formel (X) sind bekannte Synthesechemikalien.

### Verfahren (h)

Verwendet man N-(2-Acetylphenyl)-2-iodthiophen-3-carboxamid und Triphenyl(propyl)phosphoniumiodid als Ausgangsstoffe, so kann der Verlauf des erfindungsgemäßen Verfahrens (h) durch das folgende Formelschema veranschaulicht werden:

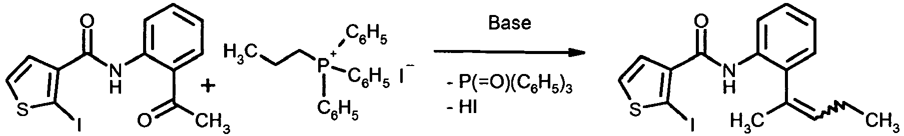

Die zur Durchführung des erfindungsgemäßen Verfahrens (h) als Ausgangsstoffe benötigten Ketone sind durch die Formel (XI) allgemein definiert. In dieser Formel haben A, Hal, R und M bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.
- G⁹: steht bevorzugt für gegebenenfalls einfach bis vierfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- G⁹: steht besonders bevorzugt für jeweils gegebenenfalls einfach bis vierfach, gleich oder verschieden durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlorund/oder Bromatomen, -SiR⁸R⁹R¹⁰, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.

Die Ketone der Formel (XI) sind noch nicht bekannt. Sie sind als neue chemische Verbindungen ebenfalls Gegenstand der vorliegenden Anmeldung. Sie werden erhalten, indem man
m) Carbonsäure-Derivate der Formel (II) 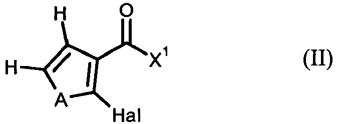 in welcher A, Hal und X¹ die oben angegebenen Bedeutungen haben,
   mit Ketoanilinen der Formel (XVII) 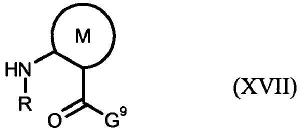 in welcher
   R, M und G⁹ die oben angegebenen Bedeutungen haben,
   gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels, umsetzt.

Die zur Durchführung des erfindungsgemäßen Verfahrens (m) als Ausgangsstoffe benötigten Carbonsäure-Derivate der Formel (II) sind bereits im Zusammenhang mit dem erfindungsgemäßen Verfahrens (a) beschrieben worden.

Die zur Durchführung des erfindungsgemäßen Verfahrens (m) weiterhin als Ausgangsstoffe benötigten Ketoaniline sind durch die Formel (XVII) allgemein definiert. In dieser Formel (XVII) haben R, M und G⁹ bevorzugt, besonders bevorzugt bzw. ganz besonders bevorzugt diejenigen Bedeutungen, die bereits im Zusammenhang mit der Beschreibung der erfindungsgemäßen Verbindungen der Formeln (I) bzw. (XI) als bevorzugt, besonders bevorzugt bzw. ganz besonders bevorzugt für diese Reste angegeben wurden.

Die Ketoaniline der Formel (XVII) sind allgemein übliche Synthesechemikalien (vgl. z.B. J. Am. Chem. Soc. 1978, 100, 4842-4857 oder US 4,032,573).

Es ist auch möglich, zunächst Hydroxyalkylanilin-Derivate der Formel (XVII-a) 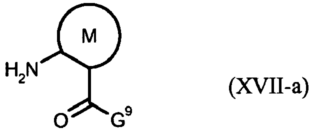 in welcher M, und G⁹ die oben angegebenen Bedeutungen haben, herzustellen und diese gegebenenfalls anschließend mit Halogeniden der Formel **Fehler! Verweisquelle konnte nicht gefunden werden.**

R^{a}-X⁶ **Fehler! Verweisquelle konnte nicht gefunden werden.**

in welcher R^{a} und X⁶ die oben angegebenen Bedeutungen haben, in Gegenwart einer Base und in Gegenwart eines Verdünnungsmittels umzusetzen. [Die Reaktionsbedingungen des erfindungsgemäßen Verfahrens (i) gelten entsprechend.]

Die zur Durchführung des erfindungsgemäßen Verfahrens (h) weiterhin als Ausgangsstoffe benötigten Phosphorverbindungen sind durch die Formel (XII) allgemein definiert.
- G¹⁰: steht bevorzugt für gegebenenfalls einfach bis vierfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₁₀-Alkyl, wobei der Cycloalkylteil seinerseits gegebenenfalls durch Halogen und/oder C₁-C₄-Alkyl substituiert sein kann.
- G¹⁰: steht besonders bevorzugt für jeweils gegebenenfalls einfach bis vierfach, gleich oder verschieden durch Fluor, Chlor, Brom, Iod, C₁-C₆-Alkylthio, C₁-C₄-Alkylsulfinyl, C₁-C₄-Alkylsulfonyl, C₁-C₄-Alkoxy, C₁-C₄-Alkylamino, Di(C₁-C₄-alkyl)amino, C₁-C₄-Halogenalkylthio, C₁-C₄-Halogenalkylsulfinyl, C₁-C₄-Halogenalkylsulfonyl, C₁-C₄-Halogenalkoxy, C₁-C₄-Halogenalkylamino, Halogen-di(C₁-C₄-alkyl)amino mit jeweils 1 bis 9 Fluor-, Chlor- und/oder Bromatomen, -SiR⁸R⁹R¹⁰, Cyclopropyl, Difluorcyclopropyl, Cyclobutyl, Cyclopentyl und/oder Cyclohexyl substituiertes, jeweils geradkettiges oder verzweigtes, jeweils an beliebiger Stelle verknüpftes Ethyl, Propyl, Butyl, Pentyl, Hexyl, Heptyl oder Octyl.
- Px: steht bevorzugt für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃.

Die Phosphorverbindungen der Formel (XII) sind bekannt und/oder können nach bekannten Verfahren hergestellt werden (vgl. z.B. Justus Liebigs Ann. Chem. 1953, 580, 44-57 oder Pure Appl. Chem. 1964, 9, 307-335).

### Reaktionsbedingungen

Als Verdünnungsmittel zur Durchführung der erfindungsgemäßen Verfahren (a), (j), (k), (l) und (m) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Die erfindungsgemäßen Verfahren (a), (j), (k), (l) und (m) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren (a), (j), (k), (l) und (m) werden gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Die erfindungsgemäßen Verfahren (a), (j), (k), (l) und (m) werden gegebenenfalls in Gegenwart eines Katalysators durchgeführt. Beispielsweise genannt seien 4-Dimethylaminopyridin, 1-Hydroxy-ben-zotriazol oder Dimethylformamid.

Die Reaktionstemperaturen können bei der Durchführung der erfindungsgemäßen Verfahren (a), (j), (k), (l) und (m) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro Mol des Carbonsäure-Derivates der Formel (II) im Allgemeinen 0,8 bis 15 Mol, vorzugsweise 0,8 bis 8 Mol an Anilin-Derivat der Formel (III) ein.

Zur Durchführung des erfindungsgemäßen Verfahrens (j) zur Herstellung der Verbindungen der Formel (IV) setzt man pro Mol des Carbonsäure-Derivates der Formel (II) im Allgemeinen 0,8 bis 15 Mol, vorzugsweise 0,8 bis 8 Mol an Halogenaniline der Formel (XIV) ein.

Zur Durchführung des erfindungsgemäßen Verfahrens (k) zur Herstellung der Verbindungen der Formel (VI) setzt man pro Mol des Carbonsäure-Derivates der Formel (II) im Allgemeinen 0,8 bis 15 Mol, vorzugsweise 0,8 bis 8 Mol an Anilinboronsäure-Derivat der Formel (XV) ein.

Zur Durchführung des erfindungsgemäßen Verfahrens (l) zur Herstellung der Verbindungen der Formel (VIII) setzt man pro Mol des Carbonsäurederivates der Formel (II) im Allgemeinen 0,8 bis 15 Mol, vorzugsweise 0,8 bis 8 Mol an Hydroxyalkylanilin-Derivat der Formel (XVI) ein.

Zur Durchführung des erfindungsgemäßen Verfahrens (m) zur Herstellung der Verbindungen der Formel (IX) setzt man pro Mol des Carbonsäure-Derivates der Formel (II) im Allgemeinen 0,8 bis 15 Mol, vorzugsweise 0,8 bis 8 Mol an Ketoanilin der Formel (XVII) ein.

Als Verdünnungsmittel zur Durchführung der erfindungsgemäßen Verfahren (b), (c) und (d) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n-oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahren (b), (c) und (d) in einem größeren Bereich variiert werden. Im allgemeinen arbeitet man bei Temperaturen von 0°C bis 180°C, vorzugsweise bei Temperaturen von 20°C bis 150°C.

Die der erfindungsgemäßen Verfahren (b), (c) und (d) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -fluoride, -phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die der erfindungsgemäßen Verfahren (b), (c) und (d) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder 1,1'-Bis(diphenylphosphino)ferrocenpalladium(II)chlorid infrage.

Es kann auch ein Palladiumkomplex in der Reaktionsmischung erzeugt werden, wenn man ein Palladiumsalz und ein Komplexligand, wie z.B. Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-tolyl)-phosphan, Natrium-3-(diphenylphosphino)benzolsulfonat, Tris-2-(Methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens (b) zur Herstellung der Verbindungen der Formel (I) setzt man pro Mol des Halogencarboxamids der Formel (IV) im Allgemeinen 1 bis 15 Mol, vorzugsweise 2 bis 8 Mol an Boronsäure-Derivaten der Formel (V) ein.

Zur Durchführung des erfindungsgemäßen Verfahrens (c) zur Herstellung der Verbindungen der Formel (I) setzt man pro Mol des Boronsäure-Derivates der Formel (VI) im Allgemeinen 0,8 bis 15 Mol, vorzugsweise 0,8 bis 8 Mol an Phenyl-Derivat der Formel (VII) ein.

Zur Durchführung des erfindungsgemäßen Verfahrens (d) zur Herstellung der Verbindungen der Formel (I) setzt man pro Mol des Halogencarboxamides der Formel (IV) im Allgemeinen 0,8 bis 15 Mol, vorzugsweise 0,8 bis 8 Mol an Phenyl-Derivat der Formel (VII) und 0,8 bis 15 Mol, vorzugsweise 0,8 bis 8 Mol an 4,4,4',4',5,5,5',5'-Octamethyl-2,2'-bis-1,3,2-dioxaborolan ein.

Als Verdünnungsmittel zur Durchführung des erfindungsgemäßen Verfahrens (e) kommen alle inerten organischen Lösungsmittel in Betracht. Hierzu gehören vorzugsweise aliphatische oder alicyclische Kohlenwasserstoffe, wie beispielsweise Petrolether, Hexan, Heptan, Cyclohexan, Methylcyclohexan oder Decalin; Ether, wie Diethylether, Diisopropylether, Methyl-t-butylether, Methyl-tert-Amylether, Dioxan, Tetrahydrofuran, 1,2-Dimethoxyethan oder 1,2-Diethoxyethan; Alkohole, wie Methanol, Ethanol, n- oder iso-Propanol, n-, iso-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren (e) wird in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Reaktionstemperaturen können bei der Durchführung des erfindungsgemäßen Verfahrens (e) in einem größeren Bereich variiert werden. Im allgemeinen arbeitet man bei Temperaturen von 0°C bis 150°C, vorzugsweise bei Temperaturen von 20°C bis 100°C.

Als Verdünnungsmittel zur Durchführung des erfindungsgemäßen Verfahrens (f) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das erfindungsgemäße Verfahren (f) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen(III)chlorid, Antimonpentachlorid, saure Ionenaustauscher, saure Tonerden und saures Kieselgel.

Die Reaktionstemperaturen können bei der Durchführung des erfindungsgemäßen Verfahrens (f) in einem größeren Bereich variiert werden. Im allgemeinen arbeitet man bei Temperaturen von 0°C bis 150°C, vorzugsweise bei Temperaturen von 0°C bis 100°C.

Als Verdünnungsmittel zur Durchführung des erfindungsgemäßen Verfahrens (g) kommen alle inerten organischen Lösungsmittel in Betracht. Hierzu gehören vorzugsweise Nitrile, wie Acetonitril, Propionitril, n- oder i-Butyronitril oder Benzonitril oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (g) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (g) wird in Gegenwart eines oder mehrerer Katalysatoren durchgeführt.

Dazu eignen sich besonders Palladiumsalze oder -komplexe. Hierzu kommen vorzugsweise Palladiumchlorid, Palladiumacetat, Tetrakis-(triphenylphosphin)-Palladium oder Bis-(triphenylphosphin)-Palladiumdichlorid infrage. Es kann auch ein Palladiumkomplex in der Reaktionsmischung erzeugt werden, wenn man ein Palladiumsalz und ein Komplexligand getrennt zur Reaktion zugibt.

Als Liganden kommen vorzugsweise Organophosphorverbindungen infrage. Beispielhaft seien genannt: Triphenylphosphin, tri-o-Tolylphosphin, 2,2'-Bis(diphenylphosphino)-1,1'-binaphthyl, Dicyclohexylphosphinebiphenyl, 1,4-Bis(diphenylphosphino)butan, Bisdiphenylphosphinoferrocen, Di-(tert-butylphosphino)biphenyl, Di(cyclohexylphosphino)biphenyl, 2-Dicyclohexylphosphino-2'-N,N-dimethylaminobiphenyl, tricyclohexylphosphine, tri-tert.-butylphosphine. Es kann aber auch auf Liganden verzichtet werden.

Das erfindungsgemäße Verfahren (g) wird ferner gegebenenfalls in Gegenwart eines weiteren Metallsalzes, wie Kupfersalzen, beispielsweise Kupfer-(I)-iodid durchgeführt.

Die Reaktionstemperaturen können bei der Durchführung des erfindungsgemäßen Verfahrens (g) in einem größeren Bereich variiert werden. Im allgemeinen arbeitet man bei Temperaturen von 20°C bis 180°C, vorzugsweise bei Temperaturen von 50°C bis 150°C.

Zur Durchführung des erfindungsgemäßen Verfahrens (g) zur Herstellung der Verbindungen der Formel (I) setzt man pro mol des Halogencarboxamides der Formel (IV) im Allgemeinen 1 bis 5 mol, vorzugsweise 1 bis 2 mol an Alkin der Formel (IX) oder Alken der Formel (X) ein.

Als Verdünnungsmittel zur Durchführung des erfindungsgemäßen Verfahrens (h) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether.

Das erfindungsgemäße Verfahren (h) wird gegebenenfalls in Gegenwart eines geeigneten Säureakzeptors durchgeführt. Als solche kommen alle üblichen starken Basen infrage. Hierzu gehören vorzugsweise Erdalkalimetall- oder Alkalimetallhydride, -hydroxide, -amide, -alkoholate oder Alkalimetall-Kohlenwasserstoffverbindungen, wie beispielsweise Natriumhydrid, Natriumhydroxid, Kaliumhydroxid, Natriumamid, Lithiumdiisopropylamid, Natrium-methylat, Natrium-ethylat, Kalium-tert.-butylat, Methyllithium, Phenyllithium oder Butyllithium.

Die Reaktionstemperaturen können bei der Durchführung des erfindungsgemäßen Verfahrens (h) in einem größeren Bereich variiert werden. Im allgemeinen arbeitet man bei Temperaturen von -80°C bis 150°C, vorzugsweise bei Temperaturen von -30°C bis 80°C.

Zur Durchführung des erfindungsgemäßen Verfahrens (h) zur Herstellung der Verbindungen der Formel (I) setzt man pro mol des Ketons der Formel (XI) im Allgemeinen 1 bis 5 mol, vorzugsweise 1 bis 2 mol an Phosphorverbindung der Formel (XII) ein.

### Reaktionsbedingungen für die Verfahren (1) bis (4) aus Schema 1:

Als Verdünnungsmittel zur Durchführung von Verfahren (1) in Schema 1 kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid; Alkohole, wie Methanol, Ethanol, iso-Propanol.

Verfahren (1) in Schema 1 wird in Gegenwart eines geeigneten Reduktionsmittels durchgeführt. Als solche kommen alle üblichen anorganischen oder organischen Reduktionsmittel infrage. Hierzu gehören vorzugsweise Erdalkalimetall- oder Alkalimetallhydride, wie z.B. Natriumhydrid, oder komplexe Hydride, wie z.B. Lithiumaluminiumhydrid, Natriumborhydrid, Natriumcyanoborhydrid, Diisobutylaluminiumhydrid, Boran, Diboran oder Borankomplexe, wie z.B. Boran-Pyridin, Silane, wie z.B. Triethylsilan, Metalle, wie z.B. Natrium, Lithium, Zink, Eisen, oder Wasserstoff.

Verfahren (1) in Schema 1 wird gegebenenfalls in Gegenwart einer geeigneten Säure oder Lewissäure durchgeführt. Als solche kommen alle üblicherweise für derartige Säure/Lewisäure vermittelten Reduktionen verwendbaren Säuren/Lewissäuren infrage. Beispielhaft genannt seien Salzsäure, Essigsäure, Trifluoressigsäure, Bortrifluorid oder komplexe Bortriluoride, wie z.B. Bortrifluoridetherat, Aluminiumtrichlorid, Certrichlorid, anorganische oder organische Titanverbindungen, wie z.B. Titantetrachlorid, Titantetraisopropylat.

Verfahren (1) in Schema 1 wird gegebenenfalls in Gegenwart eines Katalysators durchgeführt. Beispielsweise genannt seien Metalle oder Metallsalze, insbesondere Übergangsmetalle oder deren Salze, wie z.B. Platin, Palladium, Nickel (Raney-Nickel), Iridium, Rhodium, Osmium, Eisen, Ruthenium, Cobalt. Diese Metalle bzw. Metallsalze können gegebenenfalls auch an Harze oder Oberflächen bzw. Trägermaterialien (z. B. Kohle) gebunden oder aufgetragen sein.

Die Reaktionstemperaturen können bei der Durchführung von Verfahren (1) in Schema 1 in einem größeren Bereich variiert werden. Im allgemeinen arbeitet man bei Temperaturen von 0°C bis 200°C, vorzugsweise bei Temperaturen von 0°C bis 150°C.

Bei der Verwendung von Wasserstoff als Reduktionsmittel kann Verfahren (1) in Schema 1 in einem größeren Druckbereich variiert werden. Im Allgemeinen arbeitet man bei Drücken von 1 bar bis 300 bar, vorzugsweise bei 1 bar bis 100 bar.

Zur Durchführung von Verfahren (1) in Schema 1 setzt man pro Mol an Edukt im Allgemeinen 0,2 bis 10 mol, vorzugsweise 0,5 bis 5 mol an Reduktionsmittel ein.

Als Verdünnungsmittel zur Durchführung von Verfahren (2) in Schema 1 kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Verfahren (2) in Schema 1 wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Verfahren (2) in Schema 1 wird in Gegenwart eines geeigneten Halogenierungsmittels durchgeführt. Als solche kommen alle üblicherweise für derartige Halogenierungsreaktionen verwendbaren Halogenierungsmittel infrage. Beispielhaft genannt seien Halogenidbildner wie Phosgen, Phosphortribromid, Phosphortrichlorid, Phosphorpentachlorid, Phosphoroxychlorid, Oxalylchlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Brom-tripyrrolidinophosphonium-hexafluorophosphat.

Verfahren (2) in Schema 1 wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung von Verfahren (2) in Schema 1 in einem größeren Bereich variiert werden. Im allgemeinen arbeitet man bei Temperaturen von 0°C bis 200°C, vorzugsweise bei Temperaturen von 0°C bis 150°C.

Zur Durchführung von Verfahren (2) in Schema 1 setzt man pro Mol an Edukt im Allgemeinen 0,2 bis 10 mol, vorzugsweise 0,5 bis 5 mol an Halogenierungsmittel ein.

Als Verdünnungsmittel zur Durchführung von Verfahren (3) in Schema 1 kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Verfahren (3) in Schema 1 wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kaliumtert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung von Verfahren (3) in Schema 1 in einem größeren Bereich variiert werden. Im allgemeinen arbeitet man bei Temperaturen von 0°C bis 200°C, vorzugsweise bei Temperaturen von 20°C bis 150°C.

Zur Durchführung von Verfahren (3) in Schema 1 setzt man pro Mol an Edukt im Allgemeinen 0,2 bis 5 Mol, vorzugsweise 0,5 bis 2 Mol einer Verbindung der Formel H-Z^{3-C} ein.

Als Verdünnungsmittel zur Durchführung von Verfahren (4) in Schema 1 kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Verfahren (4) in Schema 1 wird in Gegenwart eines Metalls durchgeführt. Als solche kommen vorzugsweise Übergangsmetalle, wie beispielsweise Palladium, Platin, Rhodium, Nickel (Raney-Nickel), Eisen, Cobalt, Ruthenium, Iridium, Zink, oder Osmium infrage. Die Metalle können gegebenenfalls an Trägermaterialien, wie z. B. Kohle, Harze, Zeolithe, Alkali- oder Erdalkalisulfate gebunden sein.

Verfahren (4) in Schema 1 wird in Gegenwart eines Reduktionsmittels durchgeführt. Als solche kommen vorzugsweise elementarer Wasserstoff, Formiatsalze, vorzugsweise Alkaliformiatsalze, wie z.B. Natriumformiat, aber auch Ammoniumformiat oder auch Metallhydride bzw. komplexe Metallhydride, wie z.B. Lithiumaluminiumhydrid, Natriumborhydrid infrage.

Verfahren (4) in Schema 1 kann in Gegenwart von Säuren durchgeführt werden. Als solche kommen vorzugsweise organische Säuren, wie z. B. Ameisensäure, Essigsäure, Ascorbinsäure, aber auch Mineralsäuren, wie z.B. Salzsäure oder Schwefelsäure infrage.

Verfahren (4) in Schema 1 kann in Gegenwart von Basen durchgeführt werden. Als solche kommen vorzugsweise organische Basen, wie z. B. Pyridin, aber auch wässrige Lösungen von Alkali- oder Erdalkalimetallhydroxiden, wie z.B. Natriumhydroxid oder Bariumhydroxid infrage.

Die Reaktionstemperaturen können bei der Durchführung von Verfahren (4) in Schema 1 in einem größeren Bereich variiert werden. Im Allgemeinen arbeitet man bei Temperaturen von -80°C bis 300°C, vorzugsweise bei Temperaturen von 0°C bis 200°C.

Bei der Verwendung von elementarem Wasserstoff wird Verfahren (4) in Schema 1 unter einem Wasserstoffdruck zwischen 0,5 and 200 bar, bevorzugt zwischen 1 und 100 bar durchgeführt.

Zur Durchführung von Verfahren (4) in Schema 1 setzt man pro Mol an Edukt im Allgemeinen 0,8 bis 1000 Mol, vorzugsweise 1 bis 500 Mol an Reduktionsmittel (Ammoniumformiat, Hydrid etc.) ein.

### Reaktionsbedingungen für die Verfahren (5) bis (11) aus Schema 2:

Als Verdünnungsmittel zur Durchführung des Verfahrens (5) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol.

Das Verfahren (5) wird in Gegenwart einer metallorganischen Verbindung durchgeführt. Als solche kommen vorzugsweise Lithiumorganische Verbindungen, wie n-, sec-, oder tert-Butyllithium, Phenyllithium oder Methyllithium infrage.
Die Reaktionstemperaturen können bei der Durchführung des Verfahrens (5) in einem größeren Bereich variiert werden. Im allgemeinen arbeitet man bei Temperaturen von -120°C bis 100°C, vorzugsweise bei Temperaturen von -80°C bis 20°C.
Zur Durchführung des Verfahrens (5) setzt man pro Mol an Edukt im Allgemeinen 0,2 bis 5 Mol, vorzugsweise 0,5 bis 2 Mol an Ester der Formel Z^{3-E}-CO₂R¹⁷ ein.

Als Verdünnungsmittel zur Durchführung des Verfahrens (6) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Das Verfahren (6) wird in Gegenwart einer Säure durchgeführt. Als solche kommen vorzugsweise Mineralsäuren, wie z. B. Salzsäure, Iod- oder Bromwasserstoffsäure, Schwefelsäure oder auch organische Säuren, z. B. Trifluoressigsäure, Trifluormethansulfonsäure infrage.

Die Reaktionstemperaturen können bei der Durchführung des Verfahrens (6) in einem größeren Bereich variiert werden. Im allgemeinen arbeitet man bei Temperaturen von 0°C bis 300°C, vorzugsweise bei Temperaturen von 20°C bis 200°C.

Zur Durchführung des Verfahrens (6) setzt man pro Mol an Edukt im Allgemeinen 0,1 bis 10000 Mol, vorzugsweise 1 bis 2000 Mol an Säure ein.

Als Verdünnungsmittel zur Durchführung des Verfahrens (7) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Das Verfahren (7) wird in Gegenwart einer Base durchgeführt. Als solche kommen vorzugsweise Erdalkalimetall- oder Alkalimetallhydroxide, wie beispielsweise Natriumhydroxid, Kaliumhydroxid, Ammoniumhydroxid infrage.

Die Reaktionstemperaturen können bei der Durchführung des Verfahrens (7) in einem größeren Bereich variiert werden. Im allgemeinen arbeitet man bei Temperaturen von 100°C bis 300°C, vorzugsweise bei Temperaturen von 150°C bis 250°C.

Zur Durchführung des Verfahrens (7) setzt man pro Mol an Edukt im Allgemeinen 0,2 bis 5 Mol, vorzugsweise 0,5 bis 3 Mol an Hydrazin oder Hydrazinhydrat ein.

Als Verdünnungsmittel zur Durchführung des Verfahrens (8) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol.

Das Verfahren (8) wird in Gegenwart einer metallorganischen Verbindung durchgeführt. Als solche kommen vorzugsweise Methylmagnesium-chlorid, -bromid, oder -iodid oder Methyllithium infrage.

Die Reaktionstemperaturen können bei der Durchführung des Verfahrens (8) in einem größeren Bereich variiert werden. Im allgemeinen arbeitet man bei Temperaturen von -120°C bis 200°C, vorzugsweise bei Temperaturen von -80°C bis 100°C.

Zur Durchführung des Verfahrens (8) setzt man pro Mol an Edukt im Allgemeinen 0,8 bis 10 Mol, vorzugsweise 1 bis 5 Mol an metallorganischer Verbindung ein.

Als Verdünnungsmittel zur Durchführung des Verfahrens (9) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder iso-Butyronitril oder Benzonitril; Amide, wie N,N-Dimethylformamid, N,N-Dimethylacetamid, N-Methyl-formanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Das Verfahren (9) wird gegebenenfalls in Gegenwart einer Säure durchgeführt. Als solche kommen alle anorganischen und organischen Protonen- wie auch Lewissäuren, sowie auch alle polymeren Säuren infrage. Hierzu gehören beispielsweise Chlorwasserstoff, Schwefelsäure, Phosphorsäure, Ameisensäure, Essigsäure, Trifluoressigsäure, Methansulfonsäure, Trifluormethansulfonsäure, Toluolsulfonsäure, Bortrifluorid (auch als Etherat), Bortribromid, Aluminiumtrichlorid, Titantetrachlorid, Tetrabutylorthotitanat, Zinkchlorid, Eisen-III-chlorid, Antimonpentachlorid, saure Ionenaustauscher, saure Tonerden und saures Kieselgel.

Die Reaktionstemperaturen können bei der Durchführung des Verfahrens (9) in einem größeren Bereich variiert werden. Im allgemeinen arbeitet man bei Temperaturen von 0°C bis 150°C, vorzugsweise bei Temperaturen von 0°C bis 80°C.

Die Verfahren (9) und (10) können auch in einer Tandemreaktion ("Eintopf-Reaktion") durchgeführt werden.

Als Verdünnungsmittel zur Durchführung des Verfahrens (10) kommen alle inerten organischen Lösungsmittel in Betracht. Hierzu gehören vorzugsweise aliphatische oder alicyclische Kohlenwasserstoffe, wie beispielsweise Petrolether, Hexan, Heptan, Cyclohexan, Methylcyclohexan oder Decalin; Ether, wie Diethylether, Diisopropylether, Methyl-tert-butylether, Methyl-tert-Amylether, Dioxan, Tetrahydrofuran, 1,2-Dimethoxyethan oder 1,2-Diethoxyethan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.
Das Verfahren (10) wird in Gegenwart eines Katalysators durchgeführt. Als solche kommen alle Katalysatoren infrage, die für Hydrierungen üblicherweise verwendet werden. Beispielhaft seien genannt: Raney-Nickel, Palladium oder Platin, gegebenenfalls auf einem Trägermaterial, wie beispielsweise Aktivkohle.

Die Hydrierung im Verfahren (10) kann statt in Gegenwart von Wasserstoff in Kombination mit einem Katalysator auch in Anwesenheit von Triethylsilan durchgeführt werden.

Die Reaktionstemperaturen können bei der Durchführung des Verfahrens (10) in einem größeren Bereich variiert werden. Im allgemeinen arbeitet man bei Temperaturen von 0°C bis 150°C, vorzugsweise bei Temperaturen von 20°C bis 100°C.

Das Verfahren (10) wird unter einem Wasserstoffdruck zwischen 0.5 and 200 bar, bevorzugt zwischen 2 und 50 bar, besonders bevorzugt zwischen 3 und 10 bar durchgeführt.

Als Verdünnungsmittel zur Durchführung des Verfahrens (11) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-tert-Amylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid; Sulfone, wie Sulfolan; Alkohole, wie Methanol, Ethanol, n- oder iso-Propanol, n-, iso-, sec- oder tert-Butanol, Ethandiol, Propan-1,2-diol, Ethoxyethanol, Methoxyethanol, Diethylenglykolmonomethylether, Diethylenglykolmonoethylether, Triethylenglykol, deren Gemische mit Wasser oder reines Wasser.

Das Verfahren (11) wird in Gegenwart eines Metalls durchgeführt. Als solche kommen vorzugsweise Übergangsmetalle, wie beispielsweise Palladium, Platin, Rhodium, Nickel, Eisen, Cobalt, Ruthenium, Iridium oder Osmium infrage. Die Metalle können gegebenenfalls an Trägermaterialien, wie z. B. Kohle, Harze, Zeolithe, Alkali- oder Erdalkalisulfate gebunden sein.

Das Verfahren (11) wird in Gegenwart eines Reduktionsmittels durchgeführt. Als solche kommen bevorzugt elementarer Wasserstoff, Formiatsalze, vorzugsweise Alkaliformiatsalze, wie z.B. Natriumformiat, aber auch Ammoniumformiat oder auch Metallhydride (Hydrodehalogenierung) infrage.

Das Verfahren (11) kann in Gegenwart von Säuren durchgeführt werden. Als solche kommen vorzugsweise organische Säuren, wie z.B. Ameisensäure, Essigsäure, Ascorbinsäure, aber auch Mineralsäuren, wie z.B. Salzsäure oder Schwefelsäure infrage.

Das Verfahren (11) kann in Gegenwart von Basen durchgeführt werden. Als solche kommen vorzugsweise organische Basen, wie z. B. Pyridin, aber auch wässrige Lösungen von Alkali- oder Erdalkalimetallhydroxiden, wie z.B. Natriumhydroxid oder Bariumhydroxid infrage.

Die Reaktionstemperaturen können bei der Durchführung des Verfahrens (11) in einem größeren Bereich variiert werden. Im allgemeinen arbeitet man bei Temperaturen von -80°C bis 300°C, vorzugsweise bei Temperaturen von 0°C bis 200°C.

Bei der Verwendung von elementarem Wasserstoff wird das erfindungsgemäße Verfahren (11) unter einem Wasserstoffdruck zwischen 0.5 and 200 bar, bevorzugt zwischen 1 und 100 bar durchgeführt.

Zur Durchführung des erfindungsgemäßen Verfahrens (11) setzt man pro Mol an Edukt im Allgemeinen 0,8 bis 1000 Mol, vorzugsweise 1 bis 500 Mol an Reduktionsmittel (Ammoniumformiat, Hydrid etc.) ein.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides,
Rhizoctonia-Arten, wie beispielsweise Rhizoctonia solani.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanzund Saatgut, und des Bodens.

Dabei lassen sich die erfindungsgemäßen Wirkstoffe mit besonders gutem Erfolg zur Bekämpfung von Getreidekrankheiten, wie beispielsweise gegen Puccinia-Arten und von Krankheiten im Wein-, Obstund Gemüseanbau, wie beispielsweise gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehmngsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.
Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

### Fungizide:

2-Phenylphenol; 8-Hydroxychinolinsulfat; Acibenzolar-S-methyl; Aldimorph; Amidoflumet; Ampropylfos; Ampropylfos-potassium; Andoprim; Anilazine; Azaconazole; Azoxystrobin; Benalaxyl; Benalaxyl-M; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamin; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofiram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propinyl]oxy]-3-methoxyphenyl]ethyl]-3-methyl-2-[(methylsulfonyl)amino]-butanamid, 1-(1-Naphthalenyl)-1H-pyrrol-2,5-dion; 2,3,5,6-Tetrachlor-4-(methylsulfonyl)-pyridin; 2-Amino-4-methyl-Nphenyl-5-thiazolcarboxamid; 2-Chlor-N-(2,3-dihydro-1,1,3-trimethyl-1H-inden-4-yl)-3-pyridincarboxamide; 3,4,5-Trichlor-2,6-pyridindicarbonitril; Actinovate; cis-1-(4-Chlorphenyl)-2-(1H-1,2,4-triazol-1-yl)-cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyridinyl)-cyclopropancarboxamid; N-Butyl-8-(1,1-dimethyl-ethyl)-1-oxaspiro[4.5]decan-3-amin; Natriumtetrathiocarbonat; sowie Kupfersalze und -zubereitungen, wie Bordeaux mixture; Kupferhydroxid; Kupfernaphthenat; Kupferoxychlorid; Kupfersulfat; Cufraneb; Kupferoxid; Mancopper; Oxine-copper.

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* /*Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer), Tralomethrin, Transfluthrin, ZXI 8901, Pyrethrins (pyrethrum))
   2.2 Oxadiazine (z.B. Indoxacarb)
*3. Acetylcholin-Rezeptor-Agonisten*/*-Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor)
   5.2 Fiprole (z.B. Acetoprole, Ethiprole, Fipronil, Vaniliprole)
*6. Chlorid-Kanal-Aktivatoren*
   6.1 Mectine (z.B. Abamectin, Avermectin, Emamectin, Emamectin-benzoate, Ivermectin, Milbemectin, Milbemycin)
*7. Juvenilhormon-Mimetika* (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11. Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Seite-I-Elektronentransportinhibitoren*
   12.1 METI's (z.B. Fenazaquin, Fenpyroximate, Pyrimidifen, Pyridaben, Tebufenpyrad, Tolfenpyrad)
   12.2 Hydramethylnone
   12.3 Dicofol
*13. Seite-II-Elektronentransportinhibitoren*
   13.1 Rotenone
*14. Seite-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofen, Spiromesifen)
   16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Phthalamide*
   (z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-benzenedicarboxamide (CAS-Reg.-No.: 272451-65-7), Flubendiamide)
*21. Nereistoxin-Analoge*
   (z.B. Thiocyclam hydrogen oxalate, Thiosultap-sodium)
*22. Biologika, Hormone oder Pheromone*
   (z.B. Azadirachtin, Bacillus spec., Beauveria spec., Codlemone, Metarrhizium spec., Paecilomyces spec., Thuringiensin, Verticillium spec.)
*23. Wirkstoffe mit unbekannten oder nicht spezifischen Wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin,
ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/ oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den folgenden Beispielen hervor.

### Herstellungsbeispiele

### Beispiel 1

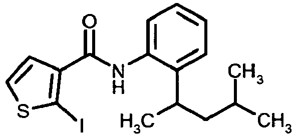

Unter Schutzgas (Argon) wird eine Lösung aus 516 mg (2.0 mmol) 2-Iod-thiophen-3-carbonsäure und_300 mg (1.7 mmol) 2-(1,3-Dimethyl-butyl)-phenylamin in 10 ml Acetonitril mit 0.56 ml (3.38 mmol) *N*,*N*-Diisopropylethylamin und 1.18 g (2.5 mmol) Bromo-tris-pyrrolidino-phosphonium hexafluorophosphat (PyBrop) versetzt. Die Reaktionsmischung wird 24 Stunden bei Raumtemperatur gerührt und zur Aufarbeitung auf Wasser gegeben, mit Essigsäureethylester extrahiert, über Natriumsulfat getrocknet und im Vakuum aufkonzentriert. Säulenchromatographie (Gradient Cyclohexan/Essigsäureethylester) liefert 150 mg (21 % der Theorie) N-[2-(1,3-Dimethylbutyl)phenyl]-2-iodthiophen-3-carboxamid [log P (pH 2.3) = 4.14].

### Beispiel 2

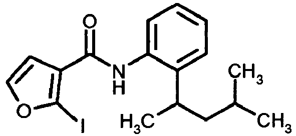

Unter Schutzgas (Argon) wird eine Lösung aus 483 mg (2.02 mmol) 2-Iod-furan-3-carbonsäure und 300 mg (1.7 mmol) 2-(1,3-Dimethyl-butyl)-phenylamin in 10 ml Acetonitril mit 0.56 ml (3.38 mmol) *N*,*N*-Diisopropylethylamin und 1.18 g (2.5 mmol) Bromo-tris-pyrrolidino-phosphonium hexafluorophosphat (PyBrop) versetzt. Die Reaktionsmischung wird 24 Stunden bei Raumtemperatur gerührt und zur Aufarbeitung auf Wasser gegeben, mit Essigsäureethylester extrahiert, über Natriumsulfat getrocknet und im Vakuum aufkonzentriert. Säulenchromatographie (Gradient Cyclohexan/Essigsäureethylester) liefert 130 mg (18 % der Theorie) N-[2-(1,3-Dimethylbutyl)phenyl]-2-iod-3-furamid [log P (pH 2.3) = 3.88].

Analog Beispiel 1 und 2, sowie entsprechend den Angaben in der allgemeinen Beschreibung der erfindungsgemäßen Herstellverfahren (a) bis (h) wurden auch die in der nachstehenden Tabelle 1 genannten Verbindungen der Formel (I) erhalten:

**Tabelle 1**

| | | | | | | |
|---|---|---|---|---|---|---|
| 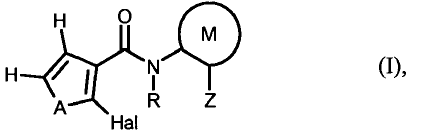 | | | | | | |

| **Nr**. | **A** | **Hal** | **R** | **M^{a)}** | **Z^{b)}** | **logP** |
|---|---|---|---|---|---|---|
| 3 | O | I | H | 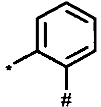 | 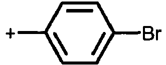 | 3.72 |
| 4 | O | I | H | 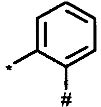 | 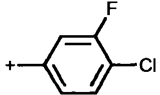 | 3.58 |
| 5 | S | I | H | 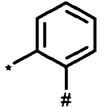 | 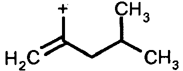 | 4.57 |
| 6 | S | I | H | 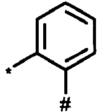 | 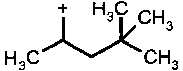 | 4.42 |
| 7 | O | I | H | 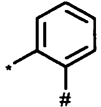 | 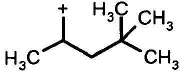 | 4.16 |
| 8 | S | I | H | 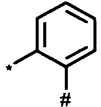 | 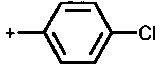 | 3.93 |
| 9 | S | I | H | 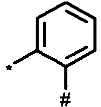 | 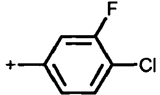 | 3.82 |
| 10 | O | I | H | 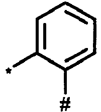 | 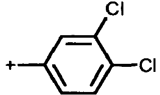 | 3.89 |
| 11 | O | I | H | 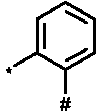 | 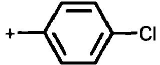 | 3.62 |
| 12 | S | I | H | 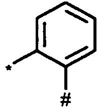 | 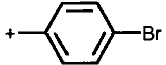 | 3.94 |
| 13 | S | I | H | 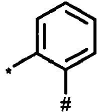 | 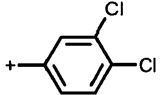 | 4.11 |
| 14 | S | I | H | 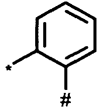 | 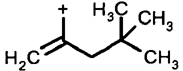 | 4.78 |
| 15 | O | I | H | 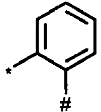 | 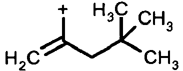 | 4.49 |
| 16 | O | I | H | 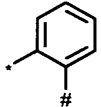 | 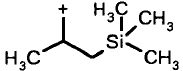 | 4.45 |
| 17 | S | I | H | 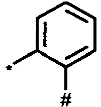 | 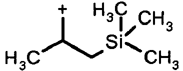 | 4.69 |
| 18 | S | I | H | 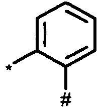 | 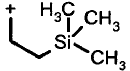 | 4.43 |
| 19 | S | I | H | 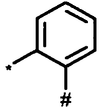 | 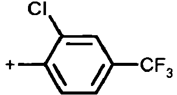 | 4.18 |
| 20 | S | I | H | 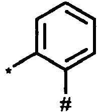 | 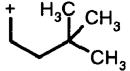 | 4.17 |
| 21 | S | I | H | 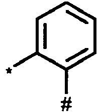 | 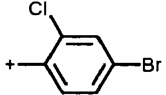 | 4.28 |
| 22 | S | I | H | 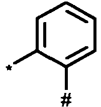 | 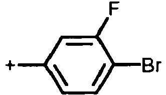 | 3.89 |
| 23 | S | I | H | 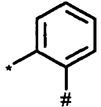 | 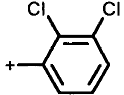 | 4.01 |
| 24 | S | I | H | 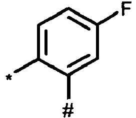 | 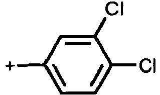 | 4.13 |
| 25 | S | I | H | 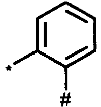 | 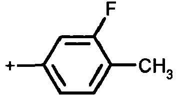 | 3.89 |
| 26 | S | I | H | 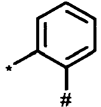 | 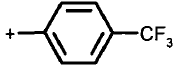 | 3.89 |
| 27 | S | Br | H | 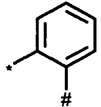 | 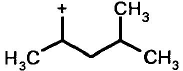 | 4.24 |
| 28 | S | Br | H | 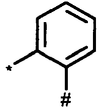 | 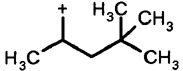 | 4.51 |
| 29 | S | Br | H | 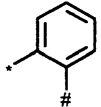 | 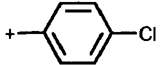 | 3.99 |
| 30 | S | Br | H | 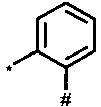 | 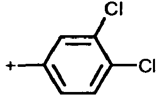 | 4.25 |
| 31 | S | Br | H | 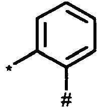 | 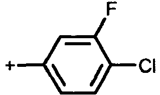 | 3.91 |
| 32 | S | I | H | 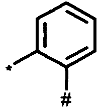 | 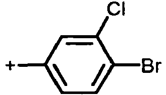 | 4,21 |
| 33 | S | I | H | 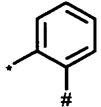 | 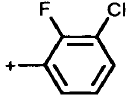 | 3,67 |
| 34 | S | I | H | 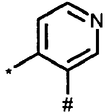 | 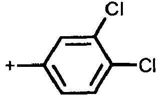 | 2,20 |
| 35 | S | I | H | 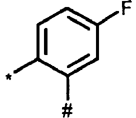 | 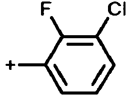 | 3,69 |
| 36 | S | I | H | 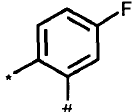 | 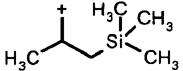 | 4,67 |
| 37 | S | I | H | 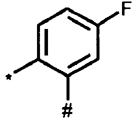 | 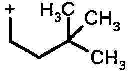 | 4,16 |
| 38 | S | I | H | 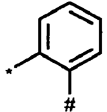 | 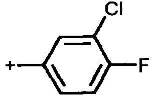 | 3,79 |
| 40 | S | I | H | 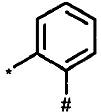 | 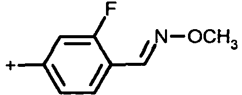 | 3,79 |
| 41 | S | I | H | 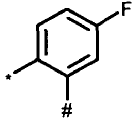 | 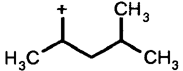 | 4,17 |
| 42 | S | I | H | 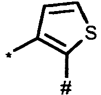 | 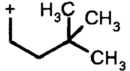 | 4,44 |
| 43 | S | I | H | 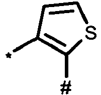 | 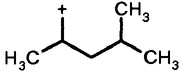 | 4,22 |
| 44 | S | Br | H | 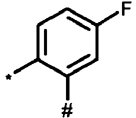 | 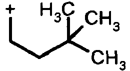 | 4,30 |
| 45 | S | Br | H | 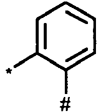 | 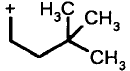 | 4,30 |

| | | | | | | |
|---|---|---|---|---|---|---|
| ^{a}) Die mit "*" markierte Bindung ist mit dem Amid, die mit "#" markierte Bindung mit dem Rest Z verknüpft. ^{b}) Die mit "+" markierte Bindung ist mit dem Rest M verknüpft. | | | | | | |

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

Eluenten für die Bestimmung im sauren Bereich (pH 2,3): 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

### Sphaerotheca-Test (Gurke) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Sphaerotheca fuliginea* inokuliert. Die Pflanzen werden dann bei ca. 23°C und einer relativen Luftfeuchtigkeit von ca. 70 % im Gewächshaus aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Sphaerotheca-Test (Gurke) / protektiv** | | |
|---|---|---|
| Wirkstoff Erfindungsgemäß | Aufwandmenge an Wirkstoff in g/ha | Wirkungsgrad in % |
| 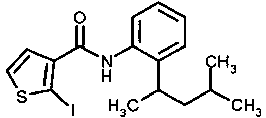 | 100 | 98 |
| 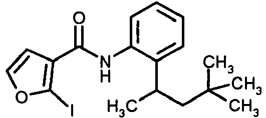 | 100 | 98 |
| 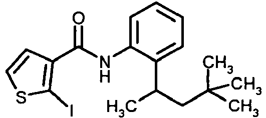 | 100 | 100 |
| 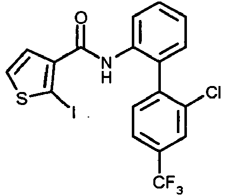 | 100 | 100 |
| 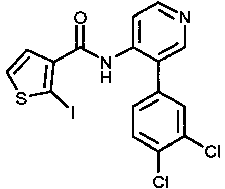 | 100 | 100 |
| 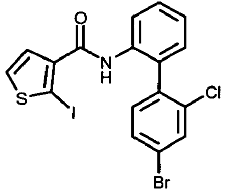 | 100 | 100 |
| 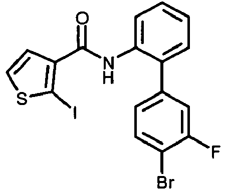 | 100 | 100 |
| 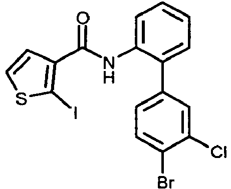 | 100 | 100 |
| 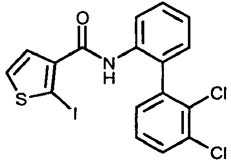 | 100 | 100 |
| 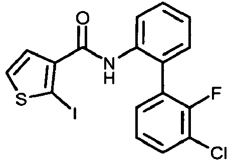 | 100 | 100 |
| 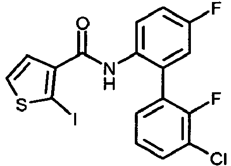 | 100 | 100 |
| 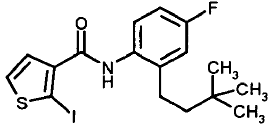 | 100 | 100 |
| 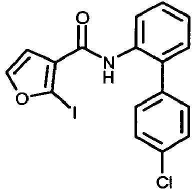 | 100 | 100 |
| 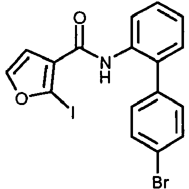 | 100 | 100 |
| 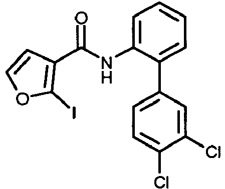 | 100 | 100 |
| 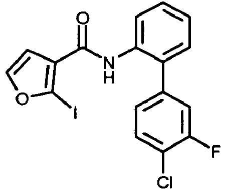 | 100 | 98 |
| 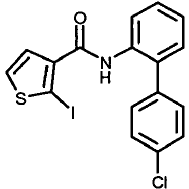 | 100 | 95 |
| 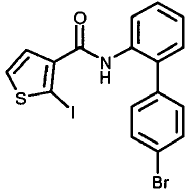 | 100 | 100 |
| 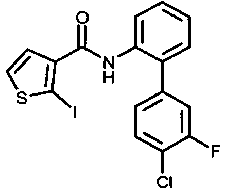 | 100 | 98 |
| 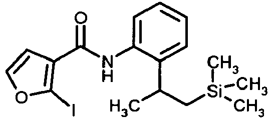 | 100 | 100 |
| 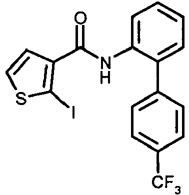 | 100 | 93 |
| 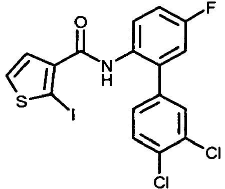 | 100 | 100 |
| 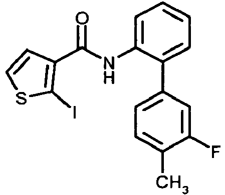 | 100 | 100 |
| 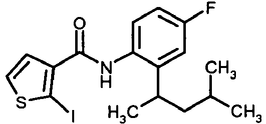 | 100 | 97 |
| 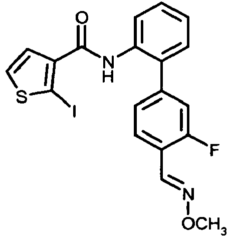 | 100 | 98 |
| 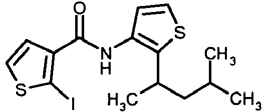 | 100 | 92 |
| 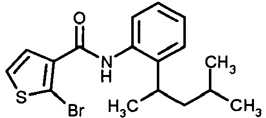 | 100 | 100 |
| 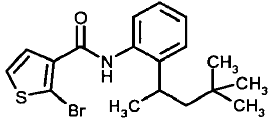 | 100 | 97 |
| 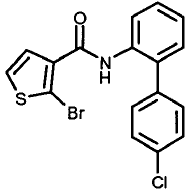 | 100 | 100 |
| 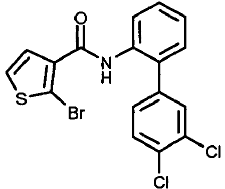 | 100 | 100 |
| 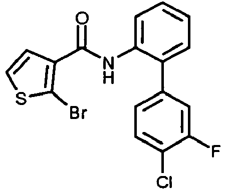 | 100 | 100 |
| 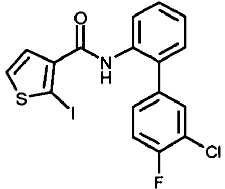 | 100 | 100 |
| 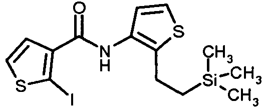 | 100 | 100 |

### Beispiel B

### Venturia - Test (Apfel) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel) / protektiv** | | |
|---|---|---|
| Wirkstoff Erfindungsgemäß | Aufwandmenge an Wirkstoff in g/ha | Wirkungsgrad in% |
| 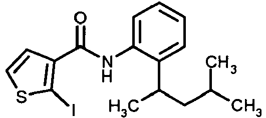 | 100 | 100 |
| 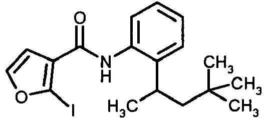 | 100 | 94 |
| 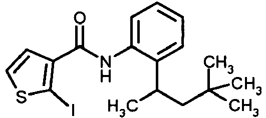 | 100 | 100 |
| 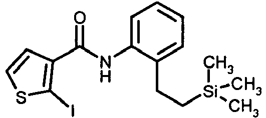 | 100 | 100 |
| 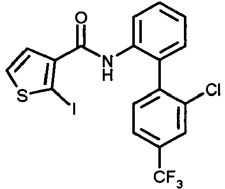 | 100 | 97 |
| 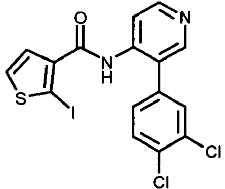 | 100 | 98 |
| 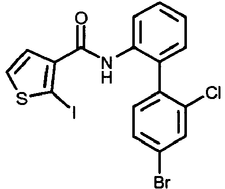 | 100 | 100 |
| 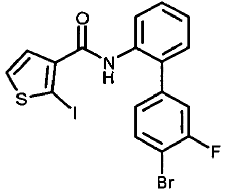 | 100 | 100 |
| 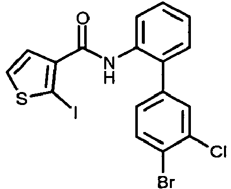 | 100 | 100 |
| 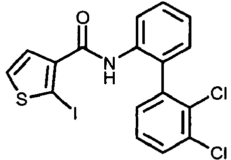 | 100 | 98 |
| 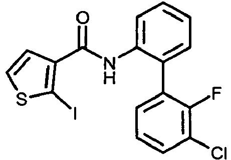 | 100 | 89 |
| 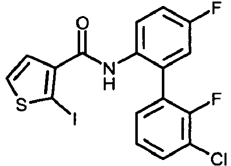 | 100 | 100 |
| 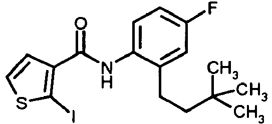 | 100 | 100 |
| 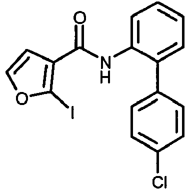 | 100 | 100 |
| 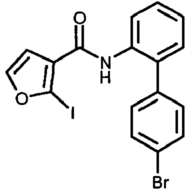 | 100 | 100 |
| 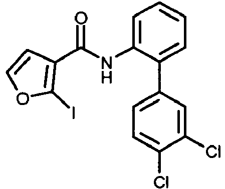 | 100 | 99 |
| 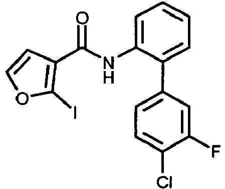 | 100 | 100 |
| 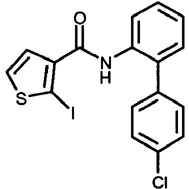 | 100 | 100 |
| 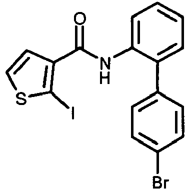 | 100 | 91 |
| 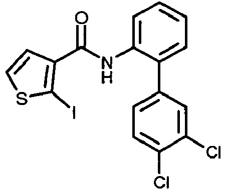 | 100 | 100 |
| 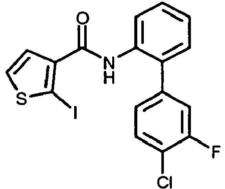 | 100 | 100 |
| 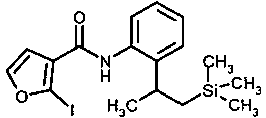 | 100 | 99 |
| 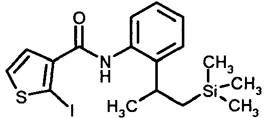 | 100 | 100 |
| 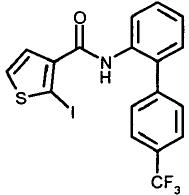 | 100 | 99 |
| 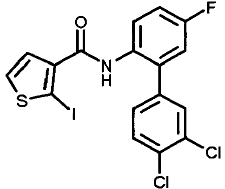 | 100 | 100 |
| 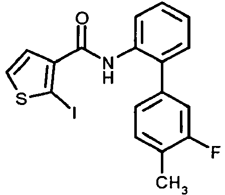 | 100 | 100 |
| 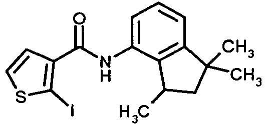 | 100 | 100 |
| 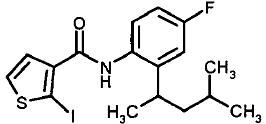 | 100 | 100 |
| 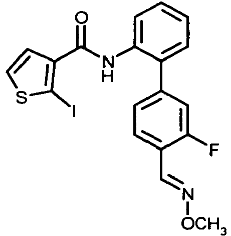 | 100 | 100 |
| 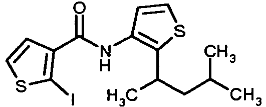 | 100 | 100 |
| 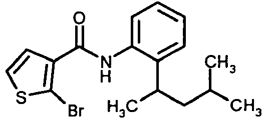 | 100 | 100 |
| 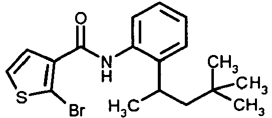 | 100 | 98 |
| 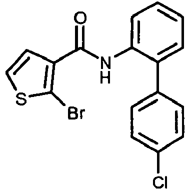 | 100 | 100 |
| 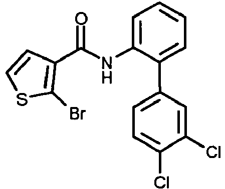 | 100 | 100 |
| 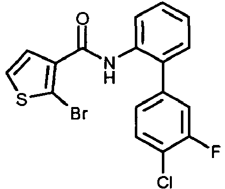 | 100 | 100 |
| 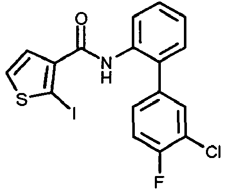 | 100 | 100 |
| 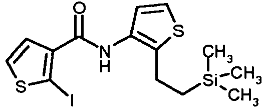 | 100 | 100 |

### Beispiel C

### Botrytis - Test (Bohne) / protektiv

| | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | |
|---|---|---|
| Wirkstoff Erfindungsgemäß | Aufwandmenge an Wirkstoff in g/ha | Wirkungsgrad in % |
| 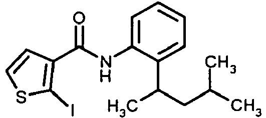 | 500 | 100 |
| 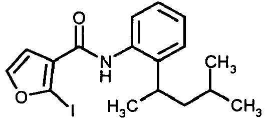 | 500 | 99 |
| 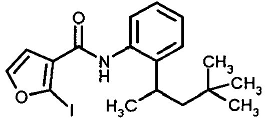 | 500 | 100 |
| 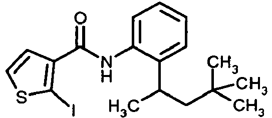 | 500 | 100 |
| 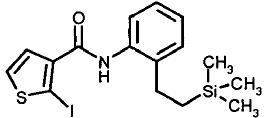 | 500 | 100 |
| 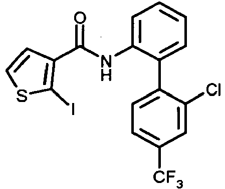 | 500 | 100 |
| 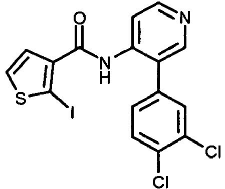 | 500 | 100 |
| 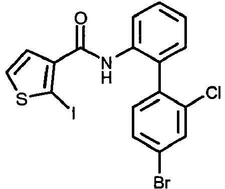 | 500 | 100 |
| 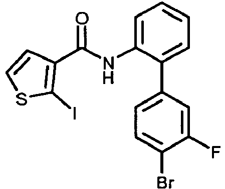 | 500 | 100 |
| 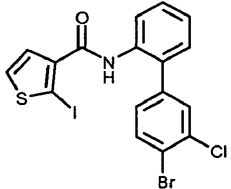 | 500 | 100 |
| 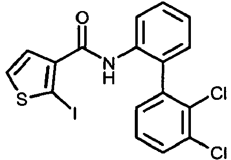 | 500 | 100 |
| 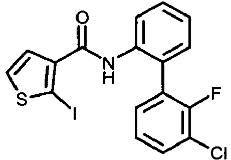 | 500 | 100 |
| 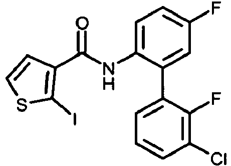 | 500 | 100 |
| 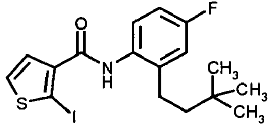 | 500 | 100 |
| 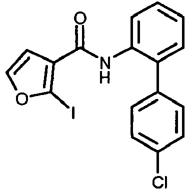 | 500 | 100 |
| 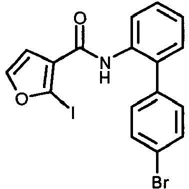 | 500 | 100 |
| 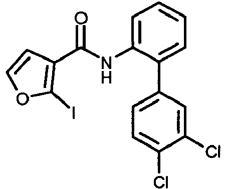 | 500 | 100 |
| 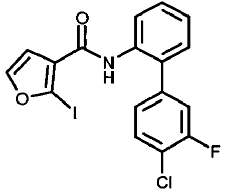 | 500 | 100 |
| 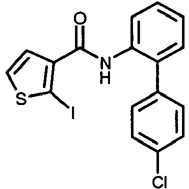 | 500 | 100 |
| 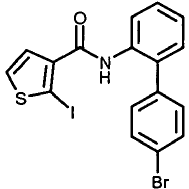 | 500 | 100 |
| 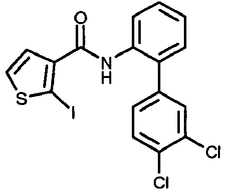 | 500 | 100 |
| 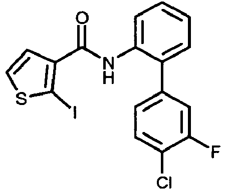 | 500 | 100 |
| 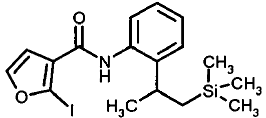 | 500 | 100 |
| 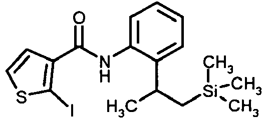 | 500 | 100 |
| 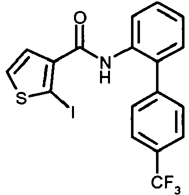 | 500 | 100 |
| 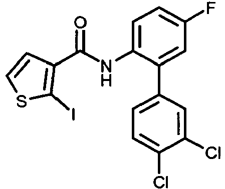 | 500 | 100 |
| 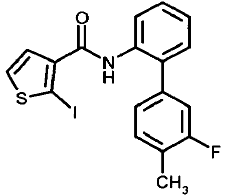 | 500 | 100 |
| 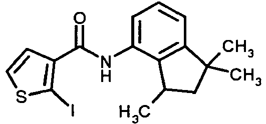 | 500 | 100 |
| 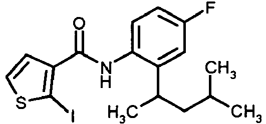 | 500 | 100 |
| 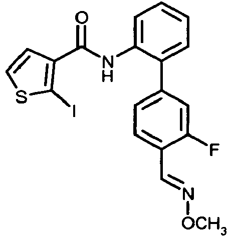 | 500 | 100 |
| 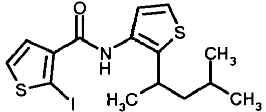 | 500 | 100 |
| 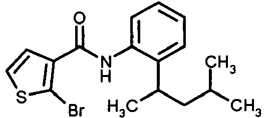 | 500 | 100 |
| 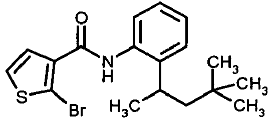 | 500 | 100 |
| 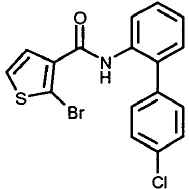 | 500 | 100 |
| 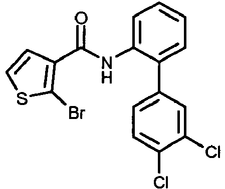 | 500 | 100 |
| 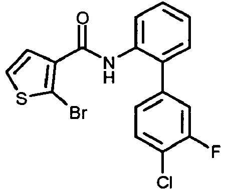 | 500 | 100 |
| 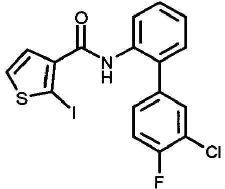 | 500 | 99 |
| 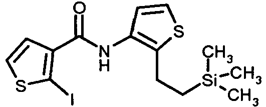 | 500 | 100 |

### Beispiel D

### Puccinia-Test (Weizen) / protektiv

| | |
|---|---|
| Lösungsmittel: | 50 Gewichtsteile N,N-Dimethylacetamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia-Test (Weizen) / protektiv** | | |
|---|---|---|
| Wirkstoff Erfindungsgemäß | Aufwandmenge an Wirkstoff in g/ha | Wirkungsgrad in % |
| 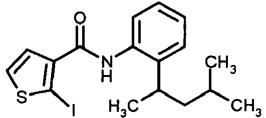 | 500 | 100 |
| 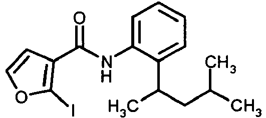 | 500 | 100 |
| 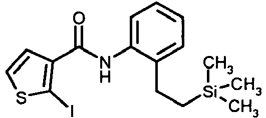 | 500 | 97 |
| 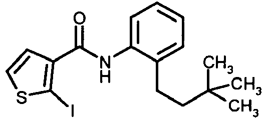 | 500 | 97 |
| 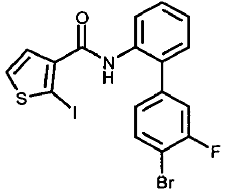 | 500 | 100 |
| 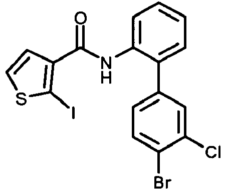 | 500 | 100 |
| 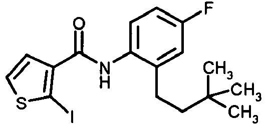 | 500 | 100 |
| 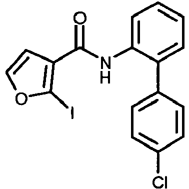 | 500 | 100 |
| 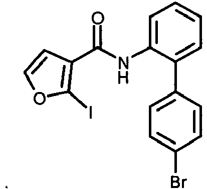 | 500 | 100 |
| 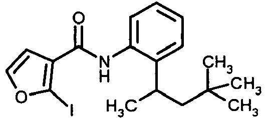 | 500 | 100 |
| 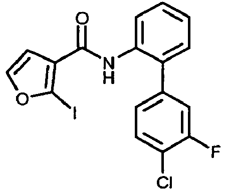 | 500 | 100 |
| 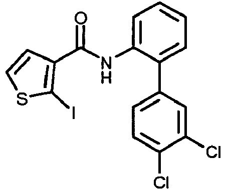 | 500 | 100 |
| 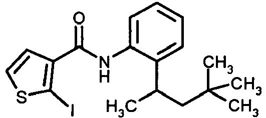 | 500 | 100 |
| 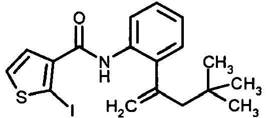 | 500 | 93 |
| 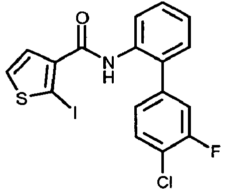 | 500 | 94 |
| 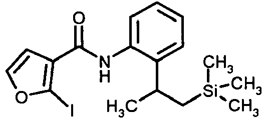 | 500 | 100 |
| 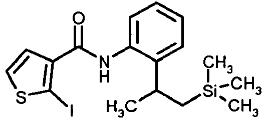 | 500 | 100 |
| 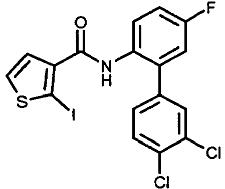 | 500 | 100 |
| 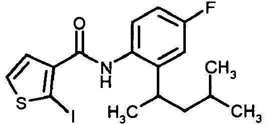 | 500 | 100 |
| 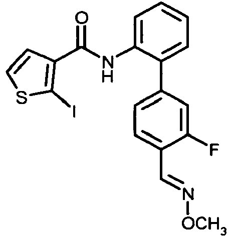 | 500 | 100 |
| 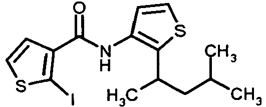 | 500 | 100 |
| 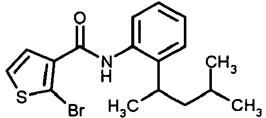 | 500 | 100 |
| 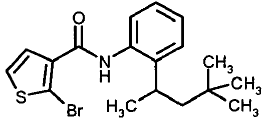 | 500 | 100 |
| 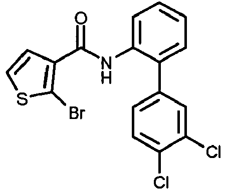 | 500 | 100 |
| 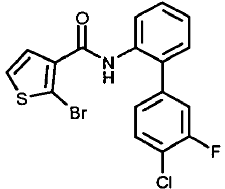 | 500 | 100 |
| 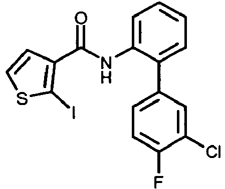 | 500 | 100 |
| 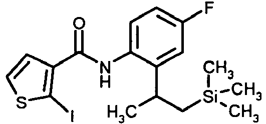 | 500 | 100 |

### Beispiel E

### Alternaria-Test (Tomate) / protektiv

| | | |
|---|---|---|
| Lösungsmittel: | 49 Gewichtsteile | N, N - Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24 Stunden bei 100 % relativer Feuchte und 20°C. Anschließend stehen die Pflanzen bei 96 % relativer Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Alternaria-Test (Tomate) / protektiv** | | |
|---|---|---|
| Wirkstoff Erfindungsgemäß | Aufwandmenge an Wirkstoff in g/ha | Wirkungsgrad in % |
| 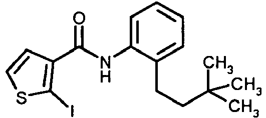 | 750 | 94 |
| 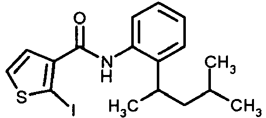 | 750 | 100 |
| 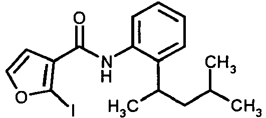 | 750 | 94 |

## Patentansprüche

1. 2-Halogenfuryl/thienyl-3-carboxamide der Formel (I) 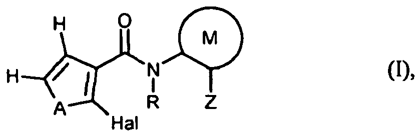 in welcher
A für O (Sauerstoff) oder S (Schwefel) steht,
Hal für Halogen steht,
R für Wasserstoff,
M für einen jeweils einfach durch R⁷ substituierten Phenyl-, Thiophen-, Pyridin-, Pyrimidin-, Pyridazin oder Pyrazin-Ring oder für einen durch R^{7-A} substituierten Thiazol-Ring steht,
R⁷ für Wasserstoff, Fluor, Chlor, Methyl, iso-Propyl, Methylthio oder Trifluormethyl steht,
R^{7-A} für Wasserstoff, Methyl, Methylthio oder Trifluormethyl steht,
Z für Z¹, Z², Z³ oder Z⁴ steht, worin
Z¹ für gegebenenfalls einfach bis fünffach, gleich oder verschieden substituiertes Phenyl steht,
Z² für jeweils gegebenenfalls einfach oder mehrfach, gleich oder verschieden substituiertes Cycloalkyl oder Bicycloalkyl steht,
Z³ für unsubstituiertes C₂-C₂₀-Alkyl oder für einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogen-alkoxy, Halogenalkylamino, Halogen-dialkylamino, -SiR⁸R⁹R¹⁰ und/oder C₃-C₆-Cycloalkyl substituiertes C₁-C₂₀-Alkyl steht, wobei der Cycloalkylteil seinerseits gegebenenfalls einfach oder mehrfach, gleich oder verschieden durch Halogen und/oder C₁-C₄-Alkyl substituiert sein kann,
Z⁴ für jeweils gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach oder mehrfach, gleich oder verschieden durch Halogen und/oder C₁-C₄-Alkyl substituiert sein kann,
R⁸ und R⁹ unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
R¹⁰ für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,

2. 2-Halogenfuryl/thienyl-3-carboxamide der Formel (I) gemäß Anspruch 1, in welcher
A für O (Sauerstoff) oder S (Schwefel) steht,
Hal für Fluor, Chlor, Brom oder Iod steht,
R für Wasserstoff,
M für einen der folgenden Cyclen steht, 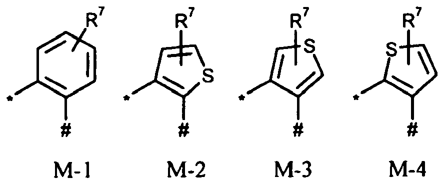 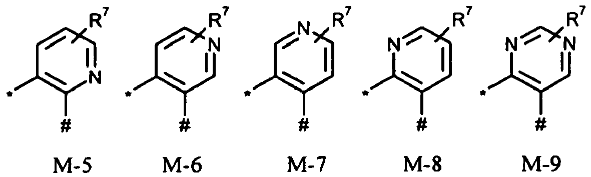 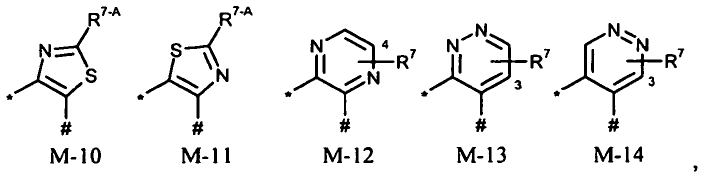 wobei die mit "*" markierte Bindung mit dem Amid, die mit "#" markierte Bindung mit dem Rest Z verknüpft ist,
R⁷ für Wasserstoff, Fluor, Chlor, Methyl, iso-Propyl, Methylthio oder Trifluormethyl steht,
R^{7-A} für Wasserstoff, Methyl oder Trifluormethyl steht,
Z für Z¹, Z², Z³ oder Z⁴ steht, worin
Z¹ für gegebenenfalls einfach bis fünffach, gleich oder verschieden substituiertes Phenyl, wobei die Substituenten jeweils aus der Liste W¹ ausgewählt sind, steht,
W¹ für Halogen, Cyano, Nitro, Amino, Hydroxy, Formyl, Carboxy, Carbamoyl, Thiocarbamoyl;
jeweils geradkettiges oder verzweigtes Alkyl, Hydroxyalkyl, Oxoalkyl, Alkoxy, Alkoxyalkyl, Alkylthioalkyl, Dialkoxyalkyl, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylaminocarbonyl, Dialkylaminocarbonyl, Arylalkylaminocarbonyl, Dialkylaminocarbonyloxy mit 1 bis 6 Kohlenstoffatomen in den jeweiligen Kohlenwasserstoffketten, Alkenylcarbonyl oder Alkinylcarbonyl, mit 2 bis 6 Kohlenstoffatomen in den jeweiligen Kohlenwasserstoffketten;
Cycloalkyl oder Cycloalkyloxy mit jeweils 3 bis 6 Kohlenstoffatomen;
jeweils gegebenenfalls einfach bis vierfach, gleich oder verschieden durch Fluor, Chlor, Oxo, Methyl, Trifluormethyl oder Ethyl substituiertes, jeweils zweifach verknüpftes Alkylen mit 3 oder 4 Kohlenstoffatomen, Oxyalkylen mit 2 oder 3 Kohlenstoffatomen oder Dioxyalkylen mit 1 oder 2 Kohlenstoffatomen;
oder die Gruppierung -C(Q¹)=N-Q², worin
Q¹ für Wasserstoff, Hydroxy oder Alkyl mit 1 bis 4 Kohlenstoffatomen, Halogen-alkyl mit 1 bis 4 Kohlenstoffatomen und 1 bis 9 Fluor-, Chlor- und/oder Bromatomen oder Cycloalkyl mit 1 bis 6 Kohlenstoffatomen steht und
Q² für Hydroxy, Amino, Methylamino, Phenyl, Benzyl oder für jeweils gegebenen-falls durch Cyano, Hydroxy, Alkoxy, Alkylthio, Alkylamino, Dialkylamino oder Phenyl substituiertes Alkyl oder Alkoxy mit 1 bis 4 Kohlenstoffatomen, oder für Alkenyloxy oder Alkinyloxy mit jeweils 2 bis 4 Kohlenstoffatomen steht, sowie jeweils gegebenenfalls im Ringteil einfach bis dreifach durch Halogen, und/ oder geradkettiges oder verzweigtes Alkyl oder Alkoxy mit 1 bis 4 Kohlenstoffatomen substituiertes Phenyl, Phenoxy, Phenylthio, Benzoyl, Benzoylethenyl, Cinnamoyl, Heterocyclyl oder Phenylalkyl, Phenylalkyloxy, Phenylalkylthio, oder Heterocyclylalkyl, mit jeweils 1 bis 3 Kohlenstoffatomen in den jeweiligen Alkylteilen, steht,
Z² für jeweils jeweils gegebenenfalls einfach bis vierfach, gleich oder verschieden durch Halogen und/oder C₁-C₄-Alkyl substituiertes Cycloalkyl oder Bicycloalkyl mit jeweils 3 bis 10 Kohlenstoffatomen steht,
Z³ für unsubstituiertes C₂-C₂₀-Alkyl oder für einfach oder mehrfach, gleich oder verschieden durch Fluor, Chlor, Brom, Iod, C₁-C₆-Alkylthio, C₁-C₆-Alkylsulfinyl, C₁-C₆-Alkyl-sulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₁-C₂₀-Alkyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann,
Z⁴ für jeweils gegebenenfalls einfach oder mehrfach, gleich oder verschieden durch Fluor, Chlor, Brom, Iod, C₁-C₆-Alkylthio, C₁-C₆-Alkylsulfinyl, C₁-C₆-Alkylsulfonyl, C₁-C₆-Alkoxy, C₁-C₆-Alkylamino, Di(C₁-C₆-alkyl)amino, C₁-C₆-Halogenalkylthio, C₁-C₆-Halogenalkylsulfinyl, C₁-C₆-Halogenalkylsulfonyl, C₁-C₆-Halogenalkoxy, C₁-C₆-Halogenalkylamino, Halogen-di(C₁-C₆-alkyl)amino, -SiR⁸R⁹R¹⁰ und/oder C₃-C₆-Cycloalkyl substituiertes C₂-C₂₀-Alkenyl oder C₂-C₂₀-Alkinyl steht, wobei der Cycloalkylteil seinerseits gegebenenfalls einfach bis vierfach, gleich oder verschieden durch Fluor, Chlor, Brom, Iod, C₁-C₄-Alkyl und/oder C₁-C₄-Halogenalkyl substituiert sein kann,
R⁸ und R⁹ unabhängig voneinander für C₁-C₆-Alkyl, C₁-C₆-Alkoxy, C₁-C₃-Alkoxy-C₁-C₃-alkyl oder C₁-C₃-Alkylthio-C₁-C₃-alkyl stehen,
R¹⁰ für C₁-C₆-Alkyl, C₁-C₆-Alkoxy, C₁-C₃-Alkoxy-C₁-C₃-alkyl, C₁-C₃-Alkylthio-C₁-C₃-alkyl, C₃-C₆-Cycloalkyl, Phenyl oder Benzyl steht,

3. Verfahren zum Herstellen der 2-Halogenfuryl/thienyl-3-carboxamide der Formel (I) gemäß Anspruch 1, **dadurch gekennzeichnet, dass** man
a) Carbonsäure-Derivate der Formel (II) 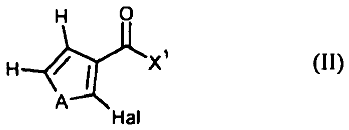 in welcher
A und Hal die in Anspruch angegebenen Bedeutungen haben und
X¹ für Halogen oder Hydroxy steht,
mit Anilin-Derivaten der Formel (III) 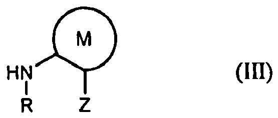 in welcher R, M und Z die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder
b) Halogencarboxamide der Formel (IV) 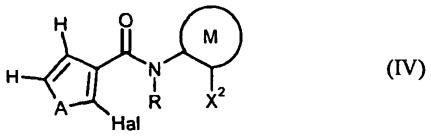 in welcher
A, Hal, R und M die in Anspruch 1 angegebenen Bedeutungen haben,
X² für Brom, Iod oder Trifluormethylsulfonat steht,
mit Boronsäure-Derivaten der Formel (V) 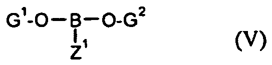 in welcher
Z¹ die in Anspruch 1 angegebenen Bedeutungen hat und
G¹ und G² jeweils für Wasserstoff oder zusammen für Tetramethylethylen stehen,
in Gegenwart eines Katalysators, gegebenenfalls in Gegenwart eines Säurebindemittels und gegebenenfalls in Gegenwart eines Verdünnungsmittels umsetzt, oder
c) Boronsäure-Derivate der Formel (VI) 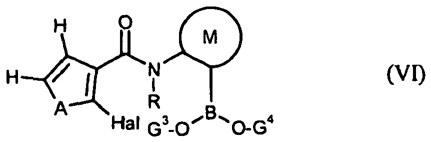 in welcher A, Hal, R und M die in Anspruch 1 angegebenen Bedeutungen haben,
G³ und G⁴ jeweils für Wasserstoff oder zusammen für Tetramethylethylen stehen, mit Phenyl-Derivaten der Formel (VII)
X³-Z¹ **Fehler! Verweisquelle konnte nicht gefunden werden.**
in welcher
Z¹ die in Anspruch 1 angegebenen Bedeutungen hat und
X³ für Chlor, Brom, Iod oder Trifluormethylsulfonat steht,
in Gegenwart eines Katalysators, gegebenenfalls in Gegenwart eines Säurebindemittels und gegebenenfalls in Gegenwart eines Verdünnungsmittels umsetzt, oder
d) Halogencarboxamide der Formel (IV) 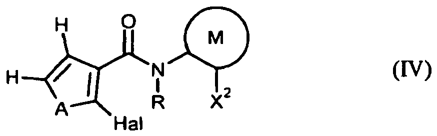 in welcher
A, Hal, R und M die in Anspruch 1 angegebenen Bedeutungen haben,
X² für Brom, Iod oder Trifluormethylsulfonat steht,
mit Phenyl-Derivaten der Formel (VII)
X³-Z¹ (VII)
in welcher
Z¹ die in Anspruch 1 angegebenen Bedeutungen hat und
X³ für Chlor, Brom, Iod oder Trifluormethylsulfonat steht,
in Gegenwart eines Palladium- oder Nickel-Katalysators und in Gegenwart von 4,4,4',4',5,5,5',5'-Octamethyl-2,2'-bis-1,3,2-dioxaborolan, gegebenenfalls in Gegenwart eines Säurebindemittels und gegebenenfalls in Gegenwart eines Verdünnungsmittels umsetzt, oder
e) 2-Halogenfuryl/thienyl-3-carboxamide der Formel (I-a) 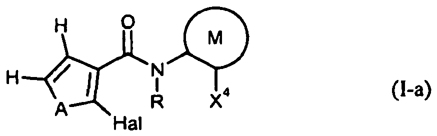 in welcher
A, Hal, R und M die in Anspruch 1 angegebenen Bedeutungen haben,
X⁴ für jeweils gegebenenfalls einfach oder mehrfach, gleich oder verschieden durch Halogen, Alkylthio, Alkylsulfinyl, Alkylsulfonyl, Alkoxy, Alkylamino, Dialkyl-amino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Alkenyl oder C₂-C₂₀-Alkinyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart eines Katalysators hydriert, oder
f) Hydroxyalkylcarboxamide der Formel (VIII) 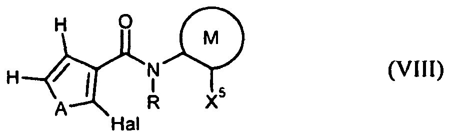 in welcher
A, Hal, R und M die in Anspruch 1 angegebenen Bedeutungen haben,
X⁵ für gegebenenfalls zusätzlich einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann,
gegebenenfalls in Gegenwart eines Verdünnungsmittels und gegebenenfalls in Gegenwart einer Säure dehydratisiert, oder
g) Halogencarboxamide der Formel (IV) 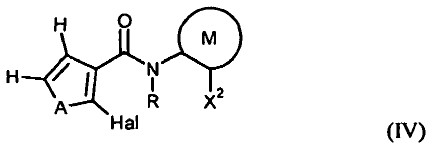 in welcher
A, Hal, R und M die in Anspruch 1 angegebenen Bedeutungen haben,
X² für Brom, Iod oder Trifluormethylsulfonat steht,
mit einem Alkin der Formel (IX) 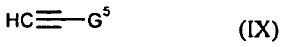 in welcher
G⁵ für gegebenenfalls einfach oder mehrfach, gleich oder verschieden durch Halogen, Alkylthio, Alkylsulfinyl, Alkylsulfonyl, Alkoxy, Alkylamino, Dialkyl-amino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₁₈-Alkyl steht, wobei der Cycloalkylteil seinerseits gegebenenfalls durch Halogen und/oder C₁-C₄-Alkyl substituiert sein kann,
oder einem Alken der Formel (X) 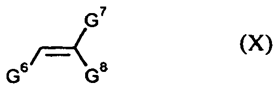 in welcher
G⁶, G⁷ und G⁸ unabhängig voneinander jeweils für Wasserstoff oder gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenal-kylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes Alkyl stehen, wobei der Cycloalkylteil seinerseits gegebenenfalls durch Halogen und/oder C₁-C₄-Alkyl substituiert sein kann und die Gesamtzahl der Kohlenstoffatome des offenkettigen Molekülteils (ohne die Substituenten) die Zahl 20 nicht übersteigt,
gegebenenfalls in Gegenwart eines Verdünnungsmittels, gegebenenfalls in Gegenwart eines Säurebindemittels und in Gegenwart eines oder mehrerer Katalysatoren umsetzt, oder
h) Ketone der Formel (XI) 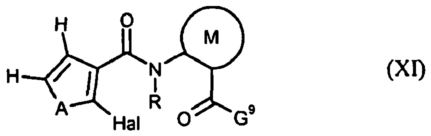 in welcher
A, Hal, R und M die in Anspruch 1 angegebenen Bedeutungen haben,
G⁹ für Wasserstoff oder gegebenenfalls einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
mit einer Phosphorverbindung der allgemeinen Formel (XII)
G¹⁰-Px (XII)
in welcher
G¹⁰ für gegebenenfalls einfach oder mehrfach, gleich oder verschieden durch Halogen, Alkylthio, Alkylsulfinyl, Alkylsulfonyl, Alkoxy, Alkylamino, Dialkyl-amino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/ oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann,
Px für eine Gruppierung -P⁺(C₆H₅)₃ Cl⁻, -P⁺(C₆H₅)₃ Br⁻, -P⁺(C₆H₅)₃ I⁻, -P(=O)(OCH₃)₃ oder -P(=O)(OC₂H₅)₃ steht,
gegebenenfalls in Gegenwart eines Verdünnungsmittels umsetzt,

4. Mittel zur Bekämpfung unerwünschter Mikroorganismen, **gekennzeichnet durch** einen Gehalt an mindestens einem 2-Halogenfuryl/thienyl-3-carboxamid der Formel (I) gemäß Anspruch 1 neben Streckmitteln und/oder oberflächenaktiven Stoffen.

5. Verwendung von 2-Halogenfuryl/thienyl-3-carboxamiden der Formel (I) gemäß Anspruch 1 zur Bekämpfung unerwünschter Mikroorganismen *sowohl im Pflanzenschutz als auch im Materialschutz.*

6. Verfahren zur Bekämpfung unerwünschter Mikroorganismen *sowohl im Pflanzenschutz als auch im Materialschutz,* **dadurch gekennzeichnet, dass** man 2-Halogenfuryl/thienyl-3-carboxamide der Formel (I) gemäß Anspruch 1 auf die Mikroorganismen und/oder deren Lebensraum ausbringt.

7. Verfahren zur Herstellung von Mitteln zur Bekämpfung unerwünschter Mikroorganismen, **dadurch gekennzeichnet, dass** man 2-Halogenfuryl/thienyl-3-carboxamide der Formel (I) gemäß Anspruch 1 mit Streckmitteln und/oder oberflächenaktiven Stoffen vermischt.

8. Halogencarboxamide der Formel (IV) 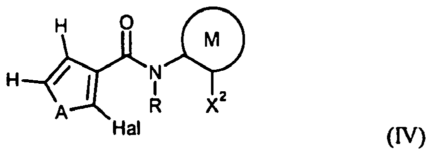 in welcher
A, Hal, R und M die in Anspruch 1 angegebenen Bedeutungen haben,
X² für Brom oder Iod steht.

9. Boronsäure-Derivate der Formel Fehler! Verweisquelle konnte nicht gefunden werden. 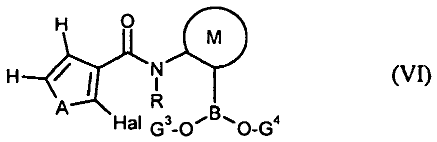 in welcher
A, Hal, R und M die in Anspruch 1 angegebenen Bedeutungen haben,
G³ und G⁴ jeweils für Wasserstoff oder zusammen für Tetramethylethylen stehen.

10. Hydroxyalkylcarboxamide der Formel (VIII) 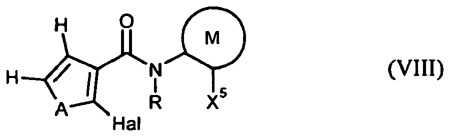 in welcher
A, Hal, R und M die in Anspruch 1 angegebenen Bedeutungen haben,
X⁵ für gegebenenfalls zusätzlich einfach oder mehrfach, gleich oder verschieden durch Halogen, Alkylthio, Alkylsulfinyl, Alkylsulfonyl, Alkoxy, Alkylamino, Dialkylamino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₂-C₂₀-Hydroxyalkyl steht, wobei der Cycloalkylteil seinerseits gegebenenfalls durch Halogen und/oder C₁-C₄-Alkyl substituiert sein kann.

11. Ketone der Formel (XI) 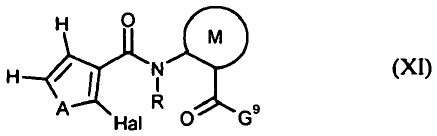 in welcher
A, Hal, R und M die in Anspruch 1 angegebenen Bedeutungen haben,
G⁹ für Wasserstoff oder gegebenenfalls einfach oder mehrfach, gleich oder verschieden durch Halogen, Alkylthio, Alkylsulfinyl, Alkylsulfonyl, Alkoxy, Alkylamino, Dialkyl-amino, Halogenalkylthio, Halogenalkylsulfinyl, Halogenalkylsulfonyl, Halogenalkoxy, Halogenalkylamino, Halogen-dialkylamino, -SiR⁸R⁹R¹⁰ und/oder C₃-C₆-Cycloalkyl substituiertes C₁-C₁₈-Alkyl steht, wobei der Cycloalkylteil seinerseits gegebenenfalls durch Halogen und/oder C₁-C₄-Alkyl substituiert sein kann.

## Claims

1. 2-Halofuryl/thienyl-3-carboxamides of the formula (I) 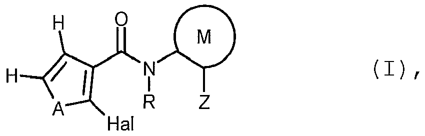 in which
A represents 0 (oxygen) or S (sulphur),
Hal represents halogen,
R represents hydrogen
M represents a phenyl, thiophene, pyridine, pyrimidine, pyridazine or pyrazine ring, each of which is monosubstituted by R⁷, or represents a thiazole ring substituted by R^{7-A},
R⁷ represents hydrogen, fluorine, chlorine, methyl, isopropyl, methylthio or trifluoromethyl,
R^{7-A} represents hydrogen, methyl, methylthio or trifluoromethyl,
Z represents Z¹, Z², Z³ or Z⁴, in which
Z¹ represents phenyl which is optionally mono- to pentasubstituted by identical or different substituents,
Z² represents bicycloalkyl or cycloalkyl, each of which is optionally mono- or polysubstituted by identical or different substituents,
Z³ represents unsubstituted C₂-C₂₀-alkyl or represents C₁-C₂₀-alkyl which is mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be mono- or polysubstituted by identical or different substituents from the group consisting of halogen and C₁-C₄-alkyl,
Z⁴ represents C₂-C₂₀-alkenyl or C₂-C₂₀-alkynyl, each of which is optionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halo-dialkylamino, -SiR⁸R⁹R¹⁰ and C₃-C₆-cycloalkyl, where the cycloalkyl moiety for its part may optionally be mono- or polysubstituted by identical or different substituents from the group consisting of halogen and C₁-C₄-alkyl,
R⁸ and R⁹ independently of one another represent hydrogen, C₁-C₈-alkyl, C₁-C₈-alkoxy, C₁-C₄-alkoxy-C₁-C₄-alkyl, C₁-C₄-alkylthio-C₁-C₄-alkyl or C₁-C₆-haloalkyl,
R¹⁰ represents hydrogen, C₁-C₈-alkyl, C₁-C₈-alkoxy, C₁-C₄-alkoxy-C₁-C₄-alkyl, C₁-C₄-alkylthio-C₁-C₄-alkyl, C₂-C₈-alkenyl, C₂-C₈-alkynyl, C₁-C₆-haloalkyl, C₂-C₆-haloalkenyl, C₂-C₆-haloalkynyl, C₃-C₆-cycloalkyl, or represents in each case optionally substituted phenyl or phenylalkyl

2. 2-Halofuryl/thienyl-3-carboxamides of the formula (I) according to Claim 1 in which
A represents 0 (oxygen) or S (sulphur),
Hal represents fluorine, chlorine, bromine or iodine,
R represents hydrogen
M represents one of the cycles below, 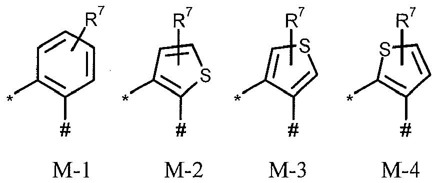 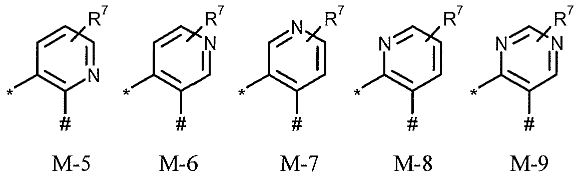 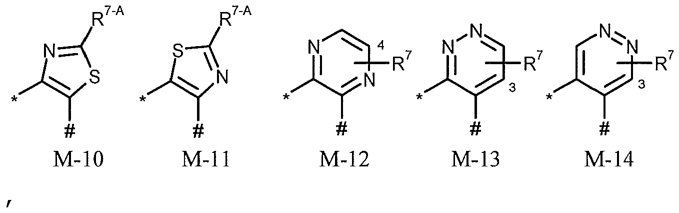 where the bond marked "*" is attached to the amide and the bond marked "#" is attached to the radical Z,
R⁷ represents hydrogen, fluorine, chlorine, methyl, isopropyl, methylthio or trifluoromethyl,
R^{7-A} represents hydrogen, methyl or trifluoromethyl,
Z represents Z¹, Z², Z³ or Z⁴, where
Z¹ represents phenyl which is optionally mono- to pentasubstituted by identical or different substituents, the substituents in each case being selected from the list W¹,
W¹ represents halogen, cyano, nitro, amino, hydroxyl, formyl, carboxy, carbamoyl, thiocarbamoyl;
in each case straight-chain or branched alkyl, hydroxyalkyl, oxoalkyl, alkoxy, alkoxyalkyl, alkylthioalkyl, dialkoxyalkyl, alkylthio, alkylsulphinyl or alkylsulphonyl having in each case 1 to 8 carbon atoms;
in each case straight-chain or branched alkenyl or alkenyloxy having in each case 2 to 6 carbon atoms;
in each case straight-chain or branched haloalkyl, haloalkoxy, haloalkylthio, halo-alkylsulphinyl or haloalkylsulphonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylaminocarbonyl, dialkylaminocarbonyl, arylalkylaminocarbonyl, dialkylaminocarbonyloxy having 1 to 6 carbon atoms in the respective hydrocarbon chains, alkenylcarbonyl or alkynylcarbonyl having 2 to 6 carbon atoms in the respective hydrocarbon chains;
cycloalkyl or cycloalkyloxy having in each case 3 to 6 carbon atoms;
doubly attached alkylene having 3 or 4 carbon atoms, oxyalkylene having 2 or 3 carbon atoms or dioxyalkylene having 1 or 2 carbon atoms, each of which is optionally mono- to tetrasubstituted by identical or different substituents from the group consisting of fluorine, chlorine, oxo, methyl, trifluoromethyl and ethyl;
or the grouping -C(Q¹)=N-Q² in which
Q¹ represents hydrogen, hydroxyl or alkyl having 1 to 4 carbon atoms, haloalkyl having 1 to 4 carbon atoms and 1 to 9 fluorine, chlorine and/or bromine atoms or cycloalkyl having 1 to 6 carbon atoms and
Q² represents hydroxyl, amino, methylamino, phenyl, benzyl or represents in each case optionally cyano-, hydroxyl-, alkoxy-, alkylthio-, alkylamino-, dialkylamino- or phenyl-substituted alkyl or alkoxy having 1 to 4 carbon atoms, or represents alkenyloxy or alkynyloxy having in each case 2 to 4 carbon atoms,
and also phenyl, phenoxy, phenylthio, benzoyl, benzoylethenyl, cinnamoyl, heterocyclyl or phenylalkyl, phenylalkyloxy, phenylalkylthio or heterocyclylalkyl having in each case 1 to 3 carbon atoms in the respective alkyl moieties, each of which radicals is optionally mono- to trisubstituted in the cyclic moiety by halogen and/or straight-chain or branched alkyl or alkoxy having 1 to 4 carbon atoms,
Z² represents cycloalkyl or bicycloalkyl having in each case 3 to 10 carbon atoms and being in each case optionally mono- to tetrasubstituted by identical or different substituents from the group consisting of halogen and/or C₁-C₄-alkyl,
Z³ represents unsubstituted C₂-C₂₀-alkyl or C₁-C₂₀-alkyl which is mono- or polysubstituted by identical or different substituents from the group consisting of fluorine, chlorine, bromine, iodine, C₁-C₆-alkylthio, C₁-C₆-alkylsulphinyl, C₁-C₆-alkylsulphonyl, C₁-C₆-alkoxy, C₁-C₆-alkylamino, di(C₁-C₆-alkyl) amino, C₁-C₆-haloalkylthio, C₁-C₆-haloalkylsulphinyl, C₁-C₆-haloalkylsulphonyl, C₁-C₆-haloalkoxy, C₁-C₆-haloalkylamino, halo-di(C₁-C₆-alkyl)amino, -SiR⁸R⁹R¹⁰ and C₃-C₆-cycloalkyl, where the cycloalkyl moiety for its part may optionally be mono- to tetrasubstituted by identical or different substituents from the group consisting of fluorine, chlorine, bromine, iodine, C₁-C₄-alkyl and C₁-C₄-haloalkyl,
Z⁴ represents C₂-C₂₀-alkenyl or C₂-C₂₀-alkynyl, each of which is optionally mono- or polysubstituted by identical or different substituents from the group consisting of fluorine, chlorine, bromine, iodine, C₁-C₆-alkylthio, C₁-C₆-alkylsulphinyl, C₁-C₆-alkylsulphonyl, C₁-C₆-alkoxy, C₁-C₆-alkylamino, di (C₁-C₆-alkyl)amino, C₁-C₆-haloalkylthio, C₁-C₆-haloalkylsulphinyl, C₁-C₆-haloalkylsulphonyl, C₁-C₆-haloalkoxy, C₁-C₆-haloalkylamino, halo-di(C₁-C₆-alkyl)amino, -SiR⁸R⁹R¹⁰ and C₃-C₆-cycloalkyl, where the cycloalkyl moiety for its part may optionally be mno- to tetrasubstituted by identical or different substituents from the group consisiting of fluorine, chlorine, bromine, iodine, C₁-C₄-alkyl and C₁-C₄-haloalkyl,
R⁸ and R⁹ independently of one another represent C₁-C₆-alkyl, C₁-C₆-alkoxy, C₁-C₃-alkoxy-C₁-C₃-alkyl or C₁-C₃-alkylthio-C₁-C₃-alkyl,
R¹⁰ represents C₁-C₆-alkyl, C₁-C₆-alkoxy, C₁-C₃-alkoxy-C₁-C₃-alkyl, C₁-C₃-alkylthio-C₁-C₃-alkyl, C₃-C₆-cycloalkyl, phenyl or benzyl.

3. Process for preparing the 2-halofuryl/thienyl-3-carboxamides of the formula (I) according to Claim 1, **characterized in that**
a) carboxylic acid derivatives of the formula (II) 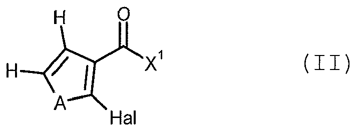 in which
A and Hal are as defined in Claim 1 and
X¹ represents halogen or hydroxyl
are reacted with aniline derivatives of the formula (III) 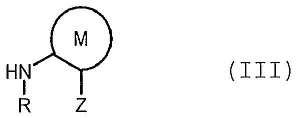 in which R, M and Z are as defined in Claim 1, if appropriate in the presence of a catalyst, if appropriate in the presence of a condensing agent, if appropriate in the presence of an acid binder and if appropriate in the presence of a diluent, or
b) halocarboxamides of the formula (IV) 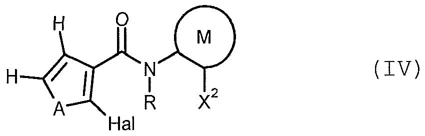 in which
A, Hal, R and M are as defined in Claim 1,
X² represents bromine, iodine or trifluoromethylsulphonate,
are reacted with boronic acid derivatives of the formula (V) 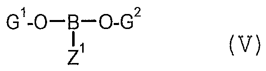 in which
Z¹ is as defined in Claim 1 and
G¹ and G² each represent hydrogen or together represent tetramethylethylene,
in the presence of a catalyst, if appropriate in the presence of an acid binder and if appropriate in the presence of a diluent, or
c) boronic acid derivatives of the formula (VI) 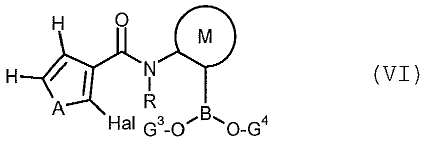 in which
A, Hal, R and M are as defined in Claim 1,
G³ and G⁴ each represent hydrogen or together represent tetramethylethylene
are reacted with phenyl derivatives of the formula (VII)
X³-Z¹ (VII)
in which
Z¹ is as defined in Claim 1 and
X³ represents chlorine, bromine, iodine or trifluoromethylsulphonate,
if appropriate in the presence of a catalyst, if appropriate in the presence of an acid binder and if appropriate in the presence of a diluent, or
d) halocarboxamides of the formula (IV) 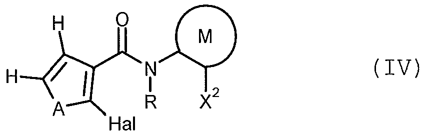 in which
A, Hal, R and M are as defined in Claim 1,
X² represents bromine, iodine or trifluoromethylsulphonate,
are reacted with phenyl derivatives of the formula (VII)
X³-Z¹ (VII)
in which
Z¹ is as defined in Claim 1 and
X³ represents chlorine, bromine, iodine or trifluoromethylsulphonate,
in the presence of a palladium or nickel catalyst and in the presence of 4,4,4',4',5,5,5',5'-octamethyl-2,2'-bis-1,3,2-dioxaborolane, if appropriate in the presence of an acid binder and if appropriate in the presence of a diluent, or
e) 2-halofuryl/thienyl-3-carboxamides of the formula (I-a) 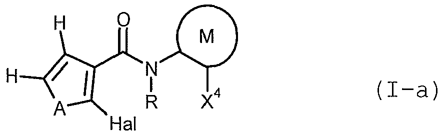 in which
A, Hal, R and M are as defined in Claim 1,
X⁴ represents C₂-C₂₀-alkenyl or C₂-C₂₀-alkynyl which are in each case optionally mono-or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be substituted by halogen and/or C₁-C₄ alkyl,
are hydrogenated, if appropriate in the presence of a diluent and if appropriate in the presence of a catalyst, or
f) hydroxyalkylcarboxamides of the formula (VIII) 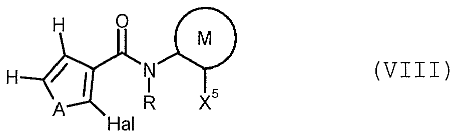 in which
A, Hal, R and M are as defined in Claim 1,
X⁵ represents C₂-C₂₀-hydroxyalkyl which is optionally additionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be substituted by halogen and/or C₁-C₄-alkyl,
are dehydrated, if appropriate in the presence of a diluent and if appropriate in the presence of an acid, or
g) halocarboxamides of the formula(IV) 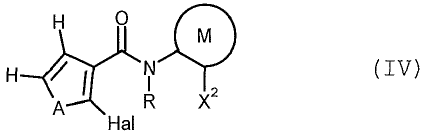 in which
A, Hal, R and M are as defined in Claim 1,
X² represents bromine, iodine or trifluoromethylsulphonate,
are reacted with an alkyne of the formula (IX)
HC≡-G⁵ (IX)
in which
G⁵ represents C₂-C₁₈-alkyl which is optionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be substituted by halogen and/or C₁-C₄-alkyl,
or an alkene of the formula (X) 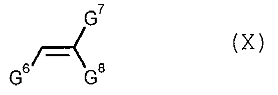 in which
G⁶, G⁷ and G⁸ independently of one another each represent hydrogen or alkyl which is optionally mono- or polysubstituted by identical or different substituents from the group consisting of halogen, alkylthio, alkylsulphinyl, alkyl-sulphonyl, alkoxy, alkylamino, dialkylamino, haloalkylthio, haloalkyl-sulphinyl, haloalkylsulphonyl, haloalkoxy, haloalkylamino, halodialkyl-amino, -SiR⁸R⁹R¹⁰ and C₃-C₆-cycloalkyl, where the cycloalkyl moiety for its part may optionally be substituted by halogen and/or C₁-C₄-alkyl and the total number of carbon atoms of the open-chain molecular moiety (without substituents) does not exceed the number 20,
if appropriate in the presence of a diluent, if appropriate in the presence of an acid binder and if appropriate in the presence of one or more catalysts, or
h) ketones of the formula (XI) 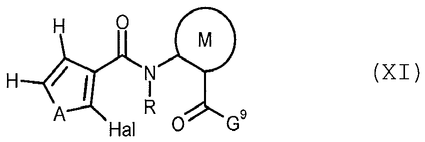 in which
A, Hal, R and M are as defined in Claim 1,
G⁹ represents hydrogen or C₁-C₁₈-alkyl which is optionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be substituted by halogen and/or C₁-C₄-alkyl,
are reacted with a phosphorus compound of the general formula (XII)
G¹⁰-Px (XII)
in which
G¹⁰ represents C₁-C₁₈-alkyl which is optionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be substituted by halogen and/or C₁-C₄-alkyl,
Px represents a grouping -P⁺(C₆H₅)₃ Cl⁻,-P⁺(C₆H₅)₃ Br⁻, -P⁺(C₆H₅)₃ I⁻, -P(=O)(OCH₃)₃ or -P(=O) (OC₂H₅)₃,
if appropriate in the presence of a diluent

4. Compositions for controlling unwanted microorganisms, **characterized in that** they comprise at least one 2-halofuryl/thienyl-3-carboxamide of the formula (I) according to Claim 1, in addition to extenders and/or surfactants.

5. Use of 2-halofuryl/thienyl-3-carboxamides of the formula (I) according to Claim 1 for controlling unwanted microorganisms both in crop protection and in the protection of materials.

6. Method for controlling unwanted microorganisms both in crop protection and in the protection of materials, **characterized in that** 2-halofuryl/thienyl-3-carboxamides of the formula (I) according to Claim 1 are applied to the microorganisms and/or their habitat.

7. Process for preparing compositions for controlling unwanted microorganisms, **characterized in that** 2-halofuryl/thienyl-3-carboxamides of the formula (I) according to Claim 1 are mixed with extenders and/or surfactants.

8. Halocarboxamides of the formula (IV) 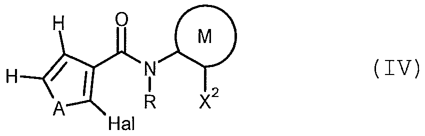 in which
A, Hal, R and M are as defined in Claim 1,
X² represents bromine or iodine.

9. Boronic acid derivatives of the formula (VI) 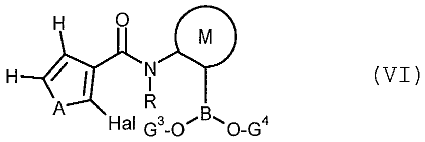 in which
A, Hal, R and M are as defined in Claim 1,
G³ and G⁴ each represent hydrogen or together represent tetramethylethylene.

10. Hydroxyalkylcarboxamides of the formula (VIII) 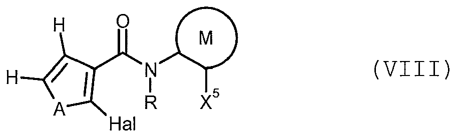 in which
A, Hal, R and M are as defined in Claim 1,
X⁵ represents C₂-C₂₀-hydroxyalkyl which is optionally additionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and/or C₃-C₆-cycloalkyl, where the cycloalkyl moiety for its part may optionally be substituted by halogen and/or C₁-C₄-alkyl.

11. Ketones of the formula (XI) 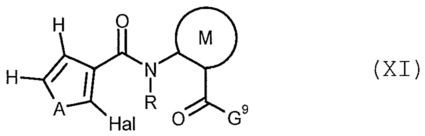 in which
A, Hal, R and M are as defined in Claim 1,
G⁹ represents hydrogen or represents C₁-C₁₈-alkyl which is optionally mono- or polysubstituted by identical or different substituents from the group consisting of halogen, alkylthio, alkylsulphinyl, alkylsulphonyl, alkoxy, alkylamino, dialkylamino, haloalkylthio, haloalkylsulphinyl, haloalkylsulphonyl, haloalkoxy, haloalkylamino, halodialkylamino, -SiR⁸R⁹R¹⁰ and C₃-C₆-cycloalkyl, where the cycloalkyl moiety for its part may optionally be substituted by halogen and/or C₁-C₄-alkyl.

## Revendications

1. 2-halogénofuryl/thiényl-3-carboxamides de formule (I) 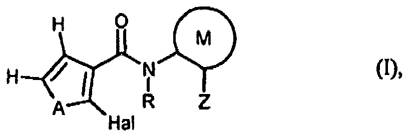 dans laquelle
A représente O (oxygène) ou S (soufre),
Hal représente halogène,
R représente hydrogène,
M représente un cycle phényle, thiophène, pyridine, pyrimidine, pyridazine ou pyrazine, chacun substitué une fois par R⁷, ou un cycle thiazole substitué par R^{7-A}, R⁷ représente hydrogène, fluor, chlore, méthyle, isopropyle, méthylthio ou trifluorométhyle,
R^{7-A} représente hydrogène, méthyle, méthylthio ou trifluorométhyle,
Z représente Z¹, Z², Z³ ou Z⁴,
Z¹ représentant phényle éventuellement substitué une à cinq fois, de manière identique ou différente,
Z² représentant cycloalkyle ou bicycloalkyle, chacun éventuellement substitué une ou plusieurs fois, de manière identique ou différente,
Z³ représentant alkyle en C₂-C₂₀ non substitué ou alkyle en C₁-C₂₀ substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogénodialkylamino, -SiR⁸R⁹R¹⁰ et/ou cycloalkyle en C₃-C₆, la partie cycloalkyle pouvant elle-même éventuellement être substituée une ou plusieurs fois, de manière identique ou différente, par halogène et/ou alkyle en C₁-C₄,
Z⁴ représentant alcényle en C₂-C₂₀ ou alcynyle en C₂-C₂₀, chacun éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogénodialkylamino, -SiR⁸R⁹R¹⁰ et/ou cycloalkyle en C₃-C₆, la partie cycloalkyle pouvant elle-même éventuellement être substituée une ou plusieurs fois, de manière identique ou différente, par halogène et/ou alkyle en C₁-C₄,
R⁸ et R⁹ représentent indépendamment l'un de l'autre hydrogène, alkyle en C₁-C₈, alcoxy en C₁-C₈, alcoxy en C₁-C₄-alkyle en C₁-C₄, alkylthio en C₁-C₄-alkyle en C₁-C₄ ou halogénoalkyle en C₁-C₆,
R¹⁰ représente hydrogène, alkyle en C₁-C₈, alcoxy en C₁-C₈, alcoxy en C₁-C₄-alkyle en C₁-C₄, alkylthio en C₁-C₄-alkyle en C₁-C₄, alcényle en C₂-C₈, alcynyle en C₂-C₈, halogénoalkyle en C₁-C₆, halogénoalcényle en C₂-C₆, halogénoalcynyle en C₂-C₆, cycloalkyle en C₃-C₆, ou phényle ou phénylalkyle, chacun éventuellement substitué.

2. 2-halogénofuryl/thiényl-3-carboxamides de formule (I) selon la revendication 1, dans lesquels
A représente O (oxygène) ou S (soufre),
Hal représente fluor, chlore, brome ou iode,
R représente hydrogène,
M représente un des cycles suivants 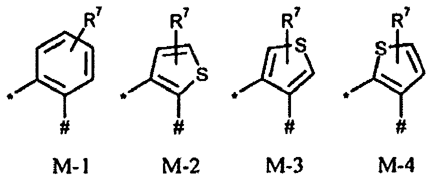 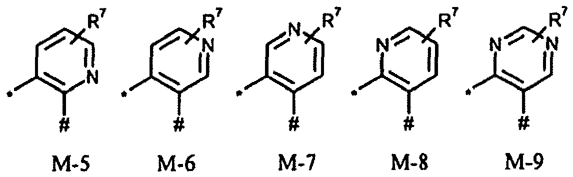 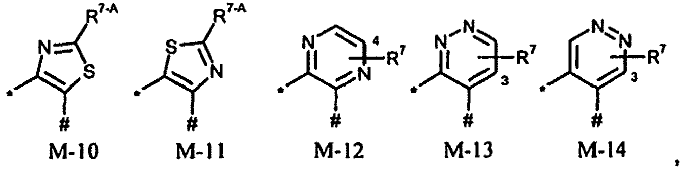 la liaison marquée avec « * » étant reliée avec l'amide, la liaison marquée avec « # » étant reliée avec le radical Z,
R⁷ représente hydrogène, fluor, chlore, méthyle, isopropyle, méthylthio ou trifluorométhyle,
R^{7-A} représente hydrogène, méthyle ou trifluorométhyle, Z représente Z¹, Z², Z³ ou Z⁴,
Z¹ représentant phényle éventuellement substitué une à cinq fois, de manière identique ou différente, les substituants étant à chaque fois choisis dans la liste W¹,
W¹ représentant halogène, cyano, nitro, amino, hydroxy, formyle, carboxy, carbamoyle, thiocarbamoyle ;
alkyle, hydroxyalkyle, oxoalkyle, alcoxy, alcoxyalkyle, alkylthioalkyle, dialcoxyalkyle, alkylthio, alkylsulfinyle ou alkylsulfonyle contenant chacun 1 à 8 atomes de carbone, chacun linéaire ou ramifié ;
alcényle ou alcényloxy contenant chacun 2 à 6 atomes de carbone, chacun linéaire ou ramifié ;
halogénoalkyle, halogénoalcoxy, halogénoalkylthio, halogénoalkylsulfinyle ou halogénoalkylsulfonyle contenant chacun 1 à 6 atomes de carbone et 1 à 13 atomes d'halogène identiques ou différents, chacun linéaire ou ramifié ;
halogénoalcényle ou halogénoalcényloxy, contenant chacun 2 à 6 atomes de carbone et 1 à 11 atomes d'halogène identiques ou différents, chacun linéaire ou ramifié ;
alkylamino, dialkylamino, alkylcarbonyle, alkylcarbonyloxy, alcoxycarbonyle, alkylaminocarbonyle, dialkylaminocarbonyle, arylalkylaminocarbonyle, dialkylaminocarbonyloxy contenant 1 à 6 atomes de carbone dans les chaînes hydrocarbonées respectives, alcénylcarbonyle ou alcynylcarbonyle, contenant 2 à 6 atomes de carbone dans les chaînes hydrocarbonées respectives, chacun linéaire ou ramifié ;
cycloalkyle ou cycloalkyloxy contenant chacun 3 à 6 atomes de carbone ;
alkylène de 3 ou 4 atomes de carbone, oxyalkylène de 2 à 3 atomes de carbone ou dioxyalkylène de 1 ou 2 atomes de carbone, chacun relié deux fois, chacun éventuellement substitué une à quatre fois, de manière identique ou différente, par fluor, chlore, oxo, méthyle, trifluorométhyle ou éthyle ;
ou le groupe -C(Q¹)=N-Q²,
Q¹ représentant hydrogène, hydroxy ou alkyle de 1 à 4 atomes de carbone, halogénoalkyle de 1 à 4 atomes de carbone et 1 à 9 atomes de fluor, chlore et/ou brome ou cycloalkyle de 1 à 6 atomes de carbone, et
Q² représentant hydroxy, amino, méthylamino, phényle, benzyle, ou alkyle ou alcoxy de 1 à 4 atomes de carbone, chacun éventuellement substitué par cyano, hydroxy, alcoxy, alkylthio, alkylamino, dialkylamino ou phényle, ou alcényloxy ou alcynyloxy chacun de 2 à 4 atomes de carbone,
ainsi que phényle, phénoxy, phénylthio, benzoyle, benzoyléthényle, cinnamoyle, hétérocyclyle ou phénylalkyle, phénylalkyloxy, phénylalkylthio ou hétérocyclylalkyle, contenant chacun 1 à 3 atomes de carbone dans les parties alkyle respectives, chacun éventuellement substitué dans la partie cyclique une à trois fois par halogène et/ou alkyle ou alcoxy linéaire ou ramifié de 1 à 4 atomes de carbone,
Z² représentant cycloalkyle ou bicycloalkyle contenant chacun 3 à 10 atomes de carbone, chacun éventuellement substitué une à quatre fois, de manière identique ou différente, par halogène et/ou alkyle en C₁-C₄,
Z³ représentant alkyle en C₂-C₂₀ non substitué ou alkyle en C₁-C₂₀ substitué une ou plusieurs fois, de manière identique ou différente, par fluor, chlore, brome, iode, alkylthio en C₁-C₆, alkylsulfinyle en C₁-C₆, alkylsulfonyle en C₁-C₆, alcoxy en C₁-C₆, alkylamino en C₁-C₆, di(alkyle en C₁-C₆)amino, halogénoalkylthio en C₁-C₆, halogénoalkylsulfinyle en C₁-C₆, halogénoalkylsulfonyle en C₁-C₆, halogénoalcoxy en C₁-C₆, halogénoalkylamino en C₁-C₆, halogéno-di(alkyle en C₁-C₆)amino, -SiR⁸R⁹R¹⁰ et/ou cycloalkyle en C₃-C₆, la partie cycloalkyle pouvant elle-même éventuellement être substituée une à quatre fois, de manière identique ou différente, par fluor, chlore, brome, iode, alkyle en C₁-C₄ et/ou halogénoalkyle en C₁-C₄,
Z⁴ représentant alcényle en C₂-C₂₀ ou alcynyle en C₂-C₂₀, chacun éventuellement substitué une ou plusieurs fois, de manière identique ou différente, par fluor, chlore, brome, iode, alkylthio en C₁-C₆, alkylsulfinyle en C₁-C₆, alkylsulfonyle en C₁-C₆, alcoxy en C₁-C₆, alkylamino en C₁-C₆, di(alkyle en C₁-C₆)amino, halogénoalkylthio en C₁-C₆, halogénoalkylsulfinyle en C₁-C₆, halogénoalkylsulfonyle en C₁-C₆, halogénoalcoxy en C₁-C₆, halogénoalkylamino en C₁-C₆, halogéno-di(alkyle en C₁-C₆)amino, -SiR⁸R⁹R¹⁰ et/ou cycloalkyle en C₂-C₆, la partie cycloalkyle pouvant elle-même éventuellement être substituée une à quatre fois, de manière identique ou différente, par fluor, chlore, brome, iode, alkyle en C₁-C₄ et/ou halogénoalkyle en C₁-C₄,
R⁸ et R⁹ représentent indépendamment l'un de l'autre alkyle en C₁-C₆, alcoxy en C₁-C₆, alcoxy en C₁-C₃-alkyle en C₁-C₃ ou alkylthio en C₁-C₃-alkyle en C₁-C₃,
R¹⁰ représente alkyle en C₁-C₆, alcoxy en C₁-C₆, alcoxy en C₁-C₃-alkyle en C₁alkylthio en C₁-C₃-alkyle en C₁-C₃, cycloalkyle en C₃-C₆, phényle ou benzyle.

3. Procédé de fabrication des 2-halogénofuryl/thiényl-3-carboxamides de formule (I) selon la revendication 1, **caractérisé en ce que**
a) des dérivés d'acide carboxylique de formule (II) 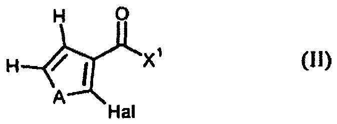 dans laquelle
A et Hal ont les significations indiquées dans la revendication 1 et
X¹ représente halogène ou hydroxy,
sont mis en réaction avec des dérivés d'aniline de formule (III) 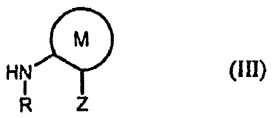 dans laquelle R, M et Z ont les significations indiquées dans la revendication 1,
éventuellement en présence d'un catalyseur, éventuellement en présence d'un agent de condensation, éventuellement en présence d'un liant d'acide et éventuellement en présence d'un diluant, ou
b) des halogénocarboxamides de formule (IV) 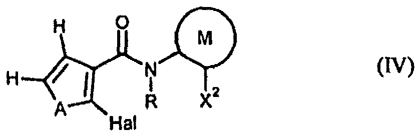 dans laquelle
A, Hal, R et M ont les significations indiquées dans la revendication 1,
X² représente brome, iode ou trifluorométhylsulfonate,
sont mis en réaction avec des dérivés de l'acide borique de formule (V) 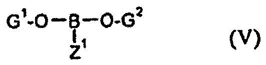 dans laquelle
Z¹ a les significations indiquées dans la revendication 1 et
G¹ et G² représentent chacun hydrogène ou ensemble tétraméthyléthylène,
en présence d'un catalyseur, éventuellement en présence d'un liant d'acide et éventuellement en présence d'un diluant, ou
c) des dérivés de l'acide borique de formule (VI) 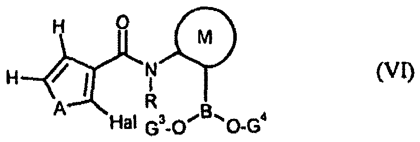 dans laquelle
A, Hal, R et M ont les significations indiquées dans la revendication 1,
G³ et G⁴ représentent chacun hydrogène ou ensemble tétraméthyléthylène,
sont mis en réaction avec des dérivés de phényle de formule (VII)
X³-Z¹ (VII)
dans laquelle
Z¹ a les significations indiquées dans la revendication 1 et
X³ représente chlore, brome, iode ou trifluorométhylsulfonate,
en présence d'un catalyseur, éventuellement en présence d'un liant d'acide et éventuellement en présence d'un diluant, ou
d) des halogénocarboxamides de formule (IV) 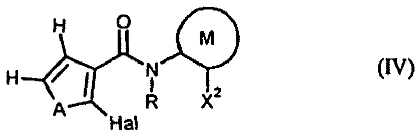 dans laquelle
A, Hal, R et M ont les significations indiquées dans la revendication 1,
X² représente brome, iode ou trifluorométhylsulfonate,
sont mis en réaction avec des dérivés de phényle de formule (VII)
X³-Z¹ (VII)
dans laquelle
Z¹ a les significations indiquées dans la revendication 1 et
X³ représente chlore, brome, iode ou trifluorométhylsulfonate,
en présence d'un catalyseur de palladium ou de nickel et en présence de 4,4,4',4',5,5,5',5'-octaméthyl-2,2'-bis-1,3,2-dioxaborolane, éventuellement en présence d'un liant d'acide et éventuellement en présence d'un diluant, ou
e) des 2-halogénofuryl/thiényl-3-carboxamides de formule (I-a) 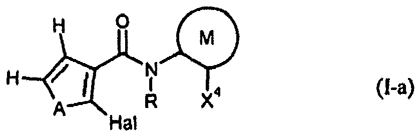 dans laquelle
A, Hal, R et M ont les significations indiquées dans la revendication 1,
X⁴ représente alcényle en C₂-C₂₀ ou alcynyle en C₂-C₂₀, chacun éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₂-C₆, la partie cycloalkyle pouvant elle-même éventuellement être substituée par halogène et/ou alkyle en C₁-C₄,
sont hydrogénés, éventuellement en présence d'un diluant et éventuellement en présence d'un catalyseur, ou
f) des hydroxyalkylcarboxamides de formule (VIII) 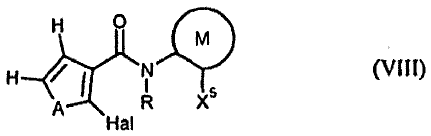 dans laquelle
A, Hal, R et M ont les significations indiquées dans la revendication 1,
X⁵ représente hydroxyalkyle en C₂-C₂₀ éventuellement en outre substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₂-C₆, la partie cycloalkyle pouvant elle-même éventuellement être substituée par halogène et/ou alkyle en C₁-C₄,
sont déshydratés, éventuellement en présence d'un diluant et éventuellement en présence d'un acide, ou
g) des halogénocarboxamides de formule (IV) 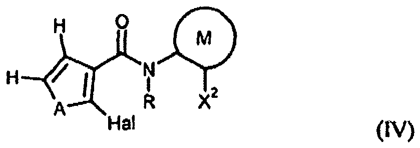 dans laquelle
A, Hal, R et M ont les significations indiquées dans la revendication 1,
X² représente brome, iode ou trifluorométhylsulfonate,
sont mis en réaction avec un alcyne de formule (IX) 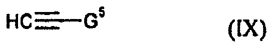 dans laquelle
G⁵ représente alkyle en C₂-C₁₈ éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₃-C₆, la partie cycloalkyle pouvant elle-même éventuellement être substituée par halogène et/ou alkyle en C₁-C₄,
ou un alcène de formule (X) 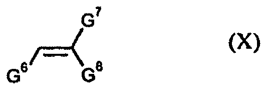 dans laquelle
G⁶, G⁷ et G⁸ représentent chacun indépendamment les uns des autres hydrogène ou alkyle éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₃-C₆, la partie cycloalkyle pouvant elle-même éventuellement être substituée par halogène et/ou alkyle en C₁-C₄, et le nombre total d'atomes de carbone de la partie à chaîne ouverte de la molécule (sans les substituants) ne dépassant pas le nombre 20,
éventuellement en présence d'un diluant, éventuellement en présence d'un liant d'acide et en présence d'un ou de plusieurs catalyseurs, ou
h) des cétones de formule (XI) 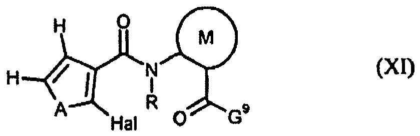 dans laquelle
A, Hal, R et M ont les significations indiquées dans la revendication 1,
G⁹ représente hydrogène ou alkyle en C₁-C₁₈ éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₂-C₆, la partie cycloalkyle pouvant elle-même éventuellement être substituée par halogène et/ou alkyle en C₁-C₄,
sont mises en réaction avec un composé de phosphore de formule générale (XII)
G¹⁰-Px (XII)
dans laquelle
G¹⁰ représente alkyle en C₁-C₁₈ éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₃-C₆, la partie cycloalkyle pouvant elle-même éventuellement être substituée par halogène et/ou alkyle en C₁-C₄,
Px représente un groupe -P⁺(C₆H₅)₃Cl⁻, -P⁺(C₆H₅)₃Br⁻, - P⁺(C₆H₅)₃I⁻, -P(=O)(OCH₃)₃ ou -P(=O)(OC₂H₅)₃, éventuellement en présence d'un diluant.

4. Agent pour lutter contre les microorganismes indésirables, **caractérisé par** une teneur en au moins un 2-halogénofuryl/thiényl-3-carboxamide de formule (I) selon la revendication 1, en plus d'extendeurs et/ou de substances tensioactives.

5. Utilisation de 2-halogénofuryl/thiényl-3-carboxamides de formule (I) selon la revendication 1 pour lutter contre les microorganismes indésirables, aussi bien dans la protection des plantes que dans la protection des matériaux.

6. Procédé de lutte contre les microorganismes indésirables, aussi bien dans la protection des plantes que dans la protection des matériaux, **caractérisé en ce que** des 2-halogénofuryl/thiényl-3-carboxamides de formule (I) selon la revendication 1 sont appliqués sur les microorganismes et/ou leur habitat.

7. Procédé de fabrication d'agents pour lutter contre des microorganismes indésirables, **caractérisé en ce que** des 2-halogénofuryl/thiényl-3-carboxamides de formule (I) selon la revendication 1 sont mélangés avec des extendeurs et/ou des substances tensioactives.

8. Halogénocarboxamides de formule (IV) 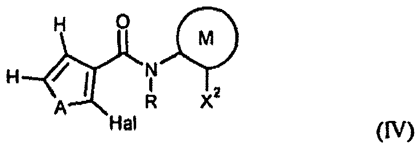 dans laquelle
A, Hal, R et M ont les significations indiquées dans la revendication 1,
X² représente brome ou iode.

9. Dérivés de l'acide borique de formule (VI) 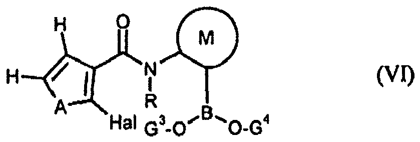 dans laquelle
A, Hal, R et M ont les significations indiquées dans la revendication 1,
G³ et G⁴ représentent chacun hydrogène ou ensemble tétraméthyléthylène.

10. Hydroxyalkylcarboxamides de formule (VIII) 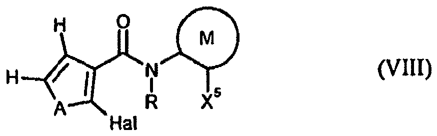 dans laquelle
A, Hal, R et M ont les significations indiquées dans la revendication 1,
X⁵ représente hydroxyalkyle en C₂-C₂₀ éventuellement en outre substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₃-C₆, la partie cycloalkyle pouvant elle-même éventuellement être substituée par halogène et/ou alkyle en C₁-C₄.

11. Cétones de formule (XI) 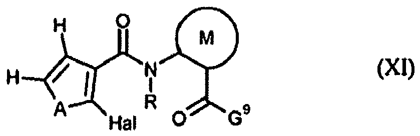 dans laquelle
A, Hal, R et M ont les significations indiquées dans la revendication 1,
G⁹ représente hydrogène ou alkyle en C₁-C₁₈ éventuellement substitué une ou plusieurs fois, de manière identique ou différente, par halogène, alkylthio, alkylsulfinyle, alkylsulfonyle, alcoxy, alkylamino, dialkylamino, halogénoalkylthio, halogénoalkylsulfinyle, halogénoalkylsulfonyle, halogénoalcoxy, halogénoalkylamino, halogéno-dialkylamino, -SiR⁸R⁹R¹⁰ et/ou cycloalkyle en C₃-C₆, la partie cycloalkyle pouvant elle-même éventuellement être substituée par halogène et/ou alkyle en C₁-C₄.
